# EUROPEAN PATENT SPECIFICATION

(11) **EP 1 879 899 B1**
(45) Date of publication and mention of the grant of the patent: **04.08.2010**
(21) Application number: 05818439.1
(22) Date of filing: 09.12.2005
(51) Int. Cl.: C07D 495/04

(54) **THIENOPYRIDINE DERIVATIVES AS POTASSIUM CHANNEL INHIBITORS**
THIENOPYRIDINDERIVATE ALS KALIUMKANALINHIBITOREN
DERIVES DE THIENOPYRIDINE EN TANT QU'INHIBITEURS DE CANAUX POTASSIQUES

(30) Priority: 09.12.2004 GB 0427005; 09.12.2004 US 634271 P
(43) Date of publication of application: 23.01.2008
(73) Proprietor: Xention Limited, Pampisford Cambridge Cambridgeshire CB2 4EF (GB)
(72) Inventor: FORD, John, Huntingdon PE28 9BN (GB); MADGE, David John, Ely, Cambridgeshire CB6 8XA (GB); PALMER,Nicholas, John, Cambridge CB1 3HJ (GB); ATHERALL, John Frederick, Essex CB10 2LL (GB); JOHN, Derek, Sawston CB2 4TA (GB)
(74) Representative: Hart, Deborah Mary
(86) International application number: PCT/GB2005/004753
(87) International publication number: WO 2006/061642

(56) References cited:
- WO-A-01/46155
- US-B1- 6 184 221
- SCHÄFER ET AL.: "2-Arylamino-thiophen-3-carbonsäurederivat e" JOURNAL F. PRAKT. CHEMIE, vol. 326, no. 6, 1984, pages 917-923, XP008061108

## Description

The present invention relates to thienopyridine compounds which are potassium channel inhibitors. Pharmaceutical compositions comprising the compounds and their use in the treatment or prevention of arrhythmias, autoimmune diseases and inflammatory diseases, including atrial fibrillation, type-2 diabetes *mellitus*, rheumatoid arthritis, type-1 diabetes, inflammatory bowel disorder and demyelinating disorders such as multiple sclerosis are also provided.

Ion channels are proteins that span the lipid bilayer of the cell membrane and provide an aqueous pathway through which specific ions such as Na⁺, K⁺, Ca²⁺ and Cl⁻ can pass (Herbert, 1998). Potassium channels represent the largest and most diverse sub-group of ion channels and they play a central role in regulating the membrane potential and controlling cellular excitability (Armstrong & Hille, 1998). Potassium channels have been categorized into gene families based on their amino acid sequence and their biophysical properties (for nomenclature see Gutman *et al*., 2003).

Compounds which modulate potassium channels have multiple therapeutic applications in several disease areas including cardiovascular, neuronal, auditory, renal, metabolic and cell proliferation (Shieh *et al.,* 2000; Ford *et al.,* 2002). More specifically potassium channels such as Kv4.3, Kit2.1, hERG, KCNQ1/minK, IKACh, IKAdo, K_{ATP} and Kv1.5 are involved in the repolarisation phase of the action potential in cardiac myocytes. These potassium channels subtypes have been associated with cardiovascular diseases and disorders including long QT syndrome, hypertrophy, ventricular fibrillation, and atrial fibrillation, all of which can cause cardiac failure and fatality (Marban, 2002).

The human delayed rectifier voltage gated potassium channel subunit, Kv1.5, is exclusively expressed in atrial myocytes and is believed to offer therapeutic opportunities for the management of atrial fibrillation for several different reasons (see review of Brendel and Peukert, 2002): (i) There is evidence that Kv1.5 underlies the cardiac ultrarapid delayed rectifier (Kv₍ᵤᵣ₎) physiological current in humans due to similar biophysical and pharmacological properties (Wang *et al.,* 1993; and Fedida *et al.,* 1993). This has been supported with antisense oligonucleotides to Kv1.5 which have been shown to reduce Kv₍ᵤᵣ₎ amplitude in human atrial myocytes (Feng *et al.,* 1997). (ii) electrophysiological recordings have demonstrated that Kv₍ᵤᵣ₎ is selectively expressed in atrial myocytes, and therefore avoids inducing potentially fatal ventricular arrhythmia through interfering with ventricular repolarisation (Amos *et al.,* 1996; Li *et al.,* 1996; and Nattel, 2002). (iii) Inhibiting Kv₍ᵤᵣ₎ in atrial fibrillation-type human atrial myocytes prolonged the action potential duration compared to normal healthy human atrial myocytes (Courtemanche *et al.,* 1999). (iv) Prolonging the action potential duration by selectively inhibiting Kv1.5 could present safer pharmacological interventions for protecting against atrial re-entrant arrhythmias such as atrial fibrillation and atrial flutter compared to traditional class III antiarrythmics, by prolonging the atrial refractory period while leaving ventricular refractoriness unaltered (Nattel *et al.,* 1999, Knobloch *et al.,* 2002; and Wirth *et al.,* 2003). Class III antiarrythmics have been widely reported as a preferred method for treating cardiac arrhythmias (Colatsky *et al.,* 1990).

Drugs that maintain the sinus rhythm long-term without proarrhythmic or other side effects are highly desirable and not currently available. Traditional and novel class III antiarrythmic potassium channel blockers have been reported to have a mechanism of action by directly modulating Kv1.5 or Kv₍ᵤᵣ₎. The known class III antiarrythmics ambasilide (Feng *et al*. , 1997), quinidine (Wang *et al.,* 1995), clofilium (Malayev *et al.,* 1995) and bertosamil (Godreau *et al.,* 2002) have all been reported as potassium channel blockers of Kv₍ᵤᵣ₎ in human atrial myocytes. The novel benzopyran derivative, NIP-142, blocks Kv1.5 channels, prolongs the atrial refractory period and terminates atrial fibrillation and flutter in *in vivo* canine models (Matsuda *et al.,* 2001), and S9947 inhibited Kv1.5 stably expressed in both Xenopus oocytes and Chinese hamster ovary (CHO) cells and Kv₍ᵤᵣ₎ in native rat and human cardiac myocytes (Bachmann *et al.,* 2001). Elsewhere, other novel potassium channel modulators which target Kv1.5 or Kv₍ᵤᵣ₎ have been described for the treatment of cardiac arrhythmias, these include biphenyls (Peukert *et al* 2003), thiophene carboxylic acid amides (WO0248131), bisaryl derivatives (WO0244137, WO0246162), carbonamide derivatives (WO0100573 WO0125189) anthranillic acid amides (WO2002100825, WO02088073, W002087568), dihydropyrimidines (WO0140231), cycloalkylamine derivatives (WO2005018635), isoquionolines (WO2005030791), quinolines (W02005030792), imidazopyrazines (W0205034837), benzopyranols (WO2005037780), isoquinolinones (WO2005046578), cycloakyl derivatives (WO03063797 indane derivatives (WO0146155 WO9804521), tetralin benzocycloheptane derivatives (WO9937607), thiazolidone and metathiazanone derivatives (WO9962891), benzamide derivatives (WO0025774), isoquinoline derivatives (WO0224655), pyridazinone derivatives (W09818475 W09818476), chroman derivatives (W09804542), benzopyran derivatives (WO0121610 WO03000675, WO0121609, W00125224, WO02064581), benzoxazine derivatives (WO0012492), and the novel compound A1998 purified from Ocean material (Xu & Xu, 2000).

Compounds that are undergoing development for atrial fibrillation have recently been reviewed (Page and Rodin, 2005).

Furthermore, the related Kv1.3 channel is expressed in both white and brown adipose tissue, and skeletal muscle (Xu *et al.,* 2004). Inhibition of the channel potentiates the hypoglycemic action of insulin, through increased insulin-stimulated glucose uptake in these tissues. This is supported by *in vivo* data, showing that Kv1.3 inhibition in mice with type-2 diabetes mellitus were significantly more sensitive to insulin. There is strong evidence that Kv1.3 inhibition improves peripheral glucose metabolism by facilitating GLUT4 translocation to the plasma membrane of adipocytes and myocytes (Desir, 2005). Small molecule inhibitors of Kv1.3 are emerging as potential targets in the management of type-2 diabetes, through their actions as insulin sensitisers (WO02-100248).

Human T-lymphocytes possess two types of potassium channels: the voltage-gated potassium Kv1.3 and the Ca2⁺-activated IKCal K⁺ channels (Leonard *et al.,* 1992, Wulff *et al.,* 2003a). These channels set the resting membrane potential of T-lymphocytes, playing a crucial role in the Ca²⁺ signal transduction pathways that lead to activation of these cells following antigenic stimulation. Disruption of these pathways can attenuate or prevent the response of T-cells to antigenic challenge resulting in immune suppression (Wulff *et al.,* 2004).

The voltage-gated Kv1.3 and the Ca²⁺-activated IKCal K⁺ channels are expressed in T-cells in distinct patterns that accompany the proliferation, maturation and differentiation of these cells. The immunomodulatory effects of channel blockers depends on the expression levels of Kv1.3 and IKCal channels, which change dramatically when T-cells transition from resting to activated cells, and during differentiation from the naïve to the memory state. Kv1.3 channels dominate functionally in quiescent cells of all T-cell subtypes (naïve, T_{CM} and T_{EM}). Activation has diametrically opposite effects on channel expression; as naïve and T_{CM} cells move from resting to proliferating blast cells, they upregulate IKCal channels. Consequently activated naïve and T_{CM} cells express -500 IKCal channels and an approximately equivalent number of Kv1.3 channels. In contrast, activation of T_{EM} cells enhances Kv1.3 expression without any change in IKCal levels. Functional Kv1.3 expression increases dramatically to 1500 Kv1.3 channels/cell, and their proliferation is sensitive to Kv1.3 blockers (Wulff *et al.,* 2003, Beeton *et al.,* 2003). B-cells also show a switch in K⁺ channel during differentiation that parallels the changes seen in the T-cell lineage (Wulff *et al.,* 2004). The discovery that the majority of myelin-reactive T-cells in patents with MS are Kv1.3^{high} T_{EM} cells, has raised interest in the therapeutic potential of Kv1.3 blockers in autoimmune disorders (Wulff *et al.,* 2003b, O'Connor *et al.,* 2001). Kv1.3 blockers have been shown to ameliorate adoptive EAE induced by myelin-specific memory T cells (a model for MS) (Beeton *et al.,* 2001) and to prevent inflammatory bone resorption in experimental periodontal disease caused mainly by memory cells (Valverde *et al.,* 2005). In addition, there is increasing evidence impiicating iate memory cells in the pathogenesis of type-1 diabetes, rheumatoid arthritis, psoriasis, inflammatory bowel disorder, Crohn's disease, Grave's disease, Plummer's disease, systemic lupus erythematosus, chronic graft rejection and chronic graft-vs-host disease (Frierich *et al.,* 2000, Yoon *et al.,* 2001, Viglietta *et al.,* 2002, Yamashita *et al.,* 2004). Specific Kv1.3 blockers might therefore constitute a new class of memory-specific immunomodulators (Shah *et al.,* 2003).

Numerous novel small molecule Kv1.3 channel blockers have been reported for the management of autoimmune disorders. These include the iminodihydroquinolines WIN173173 and CP339818 (Nguyen *et al.,* 1996), the benzhydryl piperidine UK-78,282 (Hanson *et al.* 1999), correolide (Felix *et al.,* 1999), cyclohexyl-substituted benzamide PAC (US-06194458, WO0025774), sulfamidebenzamidoindane (US-06083986), Khellinone (Baell *et al.,* 2004), dichloropenylpyrazolopyrimidine (WO-00140231) and psoralens (Wulff *et al.,* 1998., Vennekamp *et al.,* 2004, Schmitz *et al*., 2005).

Thienopyridines have been reported to be useful as antifungal agents, ligand-gated ion-channel modulators, antibacterials and enzyme inhibitors amongst others.

Thienopyridines substituted at the 2- and 3-positions by hydrogen, alkyl, cycloalkyl or aryl groups, at the 4- position by a hydroxyl group, at the 5-position by a carboxy group and by alkyl or aryl substitutents at the nitrogen of the 1-position have been claimed as potent antibacterial agents structurally related to the nalidixic acids (Gilis *et al.,* 1978).

Thienopyridines substituted at the 3-position by a phenyl group, the 2-position by a methyl ketone, the 6-position by a phenyl group, the 5-position by a nitrile group or ester and at the 4-position by an amino group have been claimed as showing antifungal activity against fungi of the family Aspergillus and to inhibit mycotoxin production (Abdelrazek *et al.,* 1992).

Thienopyridines substitiuted at the 2-, 3- and 6-positions by alkyl or aryl groups, at the 5-position by an ester, aldehyde or 3,5-dihydroxy hepterioic acid derivative and at the 4-position by a substituted phenyl group have been claimed as potent inhibitors of 3-hydroxy-3-methylglutaryl-coenzyme A (HMG-CoA) Reductase *in vitro* and to show marked cholesterol biosynthesis inhibitory activities *in vivo* (Suzuki *et al.,* 2001). Thienopyridines with a fused cycloalkyl ring between the 5- and 6-positions, and a phenyl group at the 2- and 3-positions have been shown to possess poor inhibitory activity against human acetylcholine esterase (Marco *et al*., 2002).

Thienopyridines have been claimed as anticancer agents with inhibitory action against the VEGF-2 receptor tyrosine kinase. Claimed compounds include those thienopyridines substituted at the 2-position with alkyl or aromatic moieties, unsubstituted at the 3-position and substituted at the 4-position by an amino group which may be secondary or tertiary and may be directly bound to an aromatic or heterocyclic moiety such as phenyl, indole or benzothiazole (US 6,492,383 B1, Munchof *et al., 2004* ).

Thieno[2,3-b]pyridines with a substituted aniline at the 4-position and a substituted phenyl group at the 2-position have been shown to have modest activity against the Src family of receptor tyrosine kinases as potential anticancer agents. (Boschelli *et al,* 2004)

Thieno[2,3-b]pyridines with an amino aryl or amino alkyl substituent at the 4-position, an amino group at the 3-position and a carbamoyl substituent at the 2-position have been claimed as modulators of HIV particle formation and Rev-dependant HIV production. (W02005076861).

Tricylcic 4-amino-5,6,7,8-tetrahydrothieno[2,3-b]quinoline derivatives have been claimed as agents for inhibiting acetylcholinesterase and blocking K+ channels, which is claimed to be useful for activating lowered nerve function induced by senile dementia. (JP04134083).

Thienopyridines with a carbonyl group at the 2-position and an aryl group at the 3-position have been reported as being useful in the treatment of osteoprosis (JP07076586).

4-Amino-7-hydroxy-2-methyl-5,6,7,8-tetrahydrobenzo[b]thieno[2,3-b]pyridine-3-carboxylic acid, but-2-ynyl ester (SB205384) and other tricyclic analogues has been shown to modify the GABA-A receptor modulated chloride current in rat cerebellar granule cells (Meadows *et al,* 1997).

This invention provides compounds that are potassium channel inhibitors. These compounds are particularly useful for inhibiting one or both of the potassium channels Kv1.5 (or Kv₍ᵤᵣ₎) and Kv1.3. The Kv1.5 channel is a known target for the treatment of cardiac arrhythmia in the atria such as atrial fibrillation (Nattel *et al.,* 1999; Wang *et al*., 1993); while the Kv1.3 channel is a known target for the treatment of diabetes and immunological disorders. This use is not limited to the treatment of these disorders, the compounds also being useful to treat other diseases which require potassium channel inhibition (e.g. as described in Shieh *et al.,* 2000; Ford *et al.,* 2002).

Thus, in a first aspect, the present invention provides a compound of the formula: 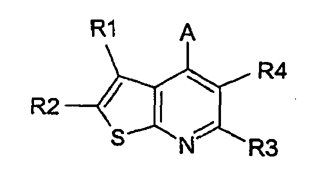 wherein
R1 is C₆-C₁₀ aryl optionally substituted by cyano, halogen, nitro, trifluoromethyl, alkyl, alkylthio, alkoxy, NR9R10, CO₂R7, C(O)NR9R10, NC(O)R7, SO₂NR9R10 or hydroxyl , 5 to 10 membered heteroaryl, optionally substituted by cyano, nitro, halogen, alkyl, alkylthio, alkoxy, NR9R10, CO₂R7, C(O)NR9R10, NC(O)R7, SO₂NR9R10 or hydroxyl or C₃-C₆ cycloalkyl, optionally substituted by halogen, cyano, nitro, NR9R10, alkoxy, hydroxyl, unsubstituted heteroaryl, CO₂R7, C(O)NR9R10. NC(O)R7 or SO₂NR9R10;
R2 is H, alkyl, nitro, CO₂R7, CONR5R6 or halo;
R3 and R4 are H, NR5R6, NC(O)R7, halo, trifluromethyl, alkyl, CONR5R6, CO₂R7, nitrile or alkoxy;
R5 and R6 may be the same or different and may be H, alkyl, aryl, heteroaryl or cycloalkyl; or R5 and R6 may together form a saturated, unsaturated or partially saturated 4 to 7 member ring, wherein said ring may optionally comprise one or more further heteroatoms selected from N, O or S;
R7 is H or alkyl;
A is halo, or a group of formula X-L-Y;
X is O, S or NR8;
R8 is H or alkyl;
L is (CH₂)ₙ, where n is 0, 1, 2, 3 or 4; and
Y is aryl, a heterocyclic group, alkyl, alkenyl or cycloalkyl;

Wherein R9 and R10 are selected from H, unsubstituted alkyl, unsubstituted aryl, unsubstituted heteroaryl, unsubstituted cycloalkyl, aminoethyl, methylaminoethyl, dimethylaminoethyl, hydroxyethyl, alkoxyethyl or R9 and R10 may together form a saturated, unsaturated or partially saturated 4 to 7 member ring;
the products of mono- and di-oxidation of sulphur and/or mono-oxidation of nitrogen moieties in compounds of formula I;
or a pharmaceutically acceptable salt thereof.

As used herein, an alkyl group or moiety is typically a linear or branched alkyl group or moiety containing from 1 to 6 carbon atoms, such as a C₁-C₄ alkyl group or moiety, for example methyl, ethyl, n-propyl, i-propyl, butyl, i-butyl and t-butyl. An alkyl group or moiety may be unsubstituted or substituted at any position. Typically, it is unsubstituted or carries one or two substituents. Suitable substituents include halogen, cyano, nitro, NR9R10, alkoxy, hydroxyl, unsubstituted aryl, unsubstituted heteroaryl, CO₂R7, C(O)NR9R10, NC(O)R7 and SO₂NR9R10.

As used herein, an aryl group is typically a C₆-C₁₀ aryl group such as phenyl or napthyl. A preferred aryl group is phenyl. An aryl group may be unsubstituted or substituted at any position. Typically, it carries 1, 2, 3 or 4 substituents. Suitable substituents include cyano, halogen, nitro, trifluoromethyl, alkyl, alkylthio, alkoxy, NR9R10, CO₂R7, C(O)NR9R10, NC(O)R7 and SO₂NR)R10 and hydroxyl.

As used herein, a heterocyclic group is a heteroaryl group, typically a 5- to 10-membered aromatic ring, such as a 5- or 6- membered ring, containing at least one heteroatom selected from O, S and N. Examples include pyridyl, pyridyl-*N*-oxide, pyrazinyl, pyrazinyl-*N*-oxide, pyrimidinyl-*N*-oxide, pyrimidinyl, pyridazinyl, pyridazinyl-*N*-oxide, furanyl, thienyl, pyrazolidinyl, pyrrolyl and pyrazolyl groups. Preferred heteroaryl groups are furanyl, thienyl and pyridyl. Examples of polycyclic heterocycles include indolyl, benzofuranyl, benzothiophenyl and benzodioxolyl. Non-aryl heterocyclic groups are also included, such as tetrahydrofuranyl or pyrrolidinyl. A heterocyclic group may be unsubstituted or substituted at any position. Suitable substituents include cyano, nitro, halogen, alkyl, alkylthio, alkoxy, NR9R10, CO₂R7, C(O)NR9R10, NC(O)R7 and SO₂NR9R10 and hydroxyl.

R9 and R10 can be the same or different, and may be selected from H, unsubstituted alkyl, unsubstituted aryl, unsubstituted heteroaryl, unsubstituted cycloalkyl, aminoethyl, methylaminoethyl, dimethylaminoethyl, hydroxyethyl, alkoxyethyl, or R9 and R10 may together form a saturated, unsaturated or partially saturated 4 to 7 member ring.

When R5 and R6 or R9 and R10 together form a saturated, unsaturated or partially saturated 4 to 7 member ring, the ring may optionally comprise one, two, or three further heteroatoms.

As used herein, alkoxy means C₁₋₃ alkoxy, cycloalkyl means C₃₋₆ cycloalkyl and halogen means Cl, F, Br, or I, preferably Cl, F or Br.

Compounds of formula I wherein mono- and di-oxidation of sulphur and/or mono-oxidation of nitrogen moieties in the compounds has taken place are also provided. Thus, compounds of formula I wherein the thieno[2,3b]pyridine moiety has been oxidized to one of the following are provided:
Thieno[2,3b]pyridine-1-oxide;
Thieno[2,3b]pyridine-1,1,-dioxide;
Thieno[2,3b]pyridine-1,1,7,-trioxide;
Thieno[2,3b]pyridine-1,7,-dioxide;
Thieno[2,3b]pyridine-7-oxide;

Preferred compounds of formula I are those wherein R1 is aryl, cycloalkyl or heteroaryl; R2 is H or alkyl; R3 and R4 are H, NR5R6, alkoxy or alkyl; X is O or NR8; R8 is H or alkyl; n is 0, 1, 2, 3 or 4 and Y is alkyl, cycloalkyl, aryl or heteroaryl. Particularly preferred compounds of formula I are those wherein R1 is aryl or heteroaryl, R2 is H or alkyl, R3 and R4 are H, NR5R6, alkoxy or alkyl, X is O or NR8, R8 is H, n is 0, 1 or 2 and Y is aryl or heteroaryl.

Preferred compounds include:
(3-Phenyl-thieno[2,3-b]pyridin-4-yl)-pyridin-2-ylmethyl-amine,
2-{3-Phenyl-4-[(pyridin-2-ylmethyl)-amino]-thieno[2,3-b]pyridin-6-ylamino}-ethanol,
2-((2-Hydroxy-ethyl)-{3-phenyl-4-[(pyridin-2-ylmethyl)-amino]-thieno[2,3-b]pyridin-6-yl}-amino)-ethanol,
(6-Chloro-3-phenyl-thieno[2,3-b]pyridin-4-yl)-pyridin-2-ylmethyl-amine,
[3-(4-Fluoro-phenyl)-thieno[2,3-b]pyridin-4-yl]-pyridin-2-ylmethyl-amine,
2-[{3-(4-Fluoro-phenyl)-4-[(pyridin-2-ylmethyl)-amino]-thieno[2,3-b]pyridin-6-yl}-(2-hydroxy-ethyl)-amino]-ethanol,
2-[{3-(4-Fluoro-phenyl)-4-[(6-methyl-pyridin-2-ylmethyl)-amino]-thieno[2,3-b]pyridin-6-yl}-(2-hydroxy-ethyl)-amino]-ethanol,
2-{3-(4-Fluoro-phenyl)-4-[(pyridin-2-ylmethyl)-amino]-thieno[2,3-b]pyridin-6-ylamino}-ethanol,
2-{3-(4-Fluoro-phenyl)-4-[(6-methyl-pyridin-2-ylmethyl)-amino]-thieno[2,3-b]pyridin-6-ylamino}-ethanol,
2-{2-Methyl-3-phenyl-4-[(pyridin-2-ylmethyl)-amino]-thieno[2,3-b]pyridin-6-ylamino}-ethanol,
[6-Chloro-3-(4-fluoro-phenyl)-thieno[2,3-b]pyridin-4-yl]-pyridin-2-ylmethyl-amine,
[6-Chloro-3-(4-fluoro-phenyl)-thieno[2,3-b]pyridin-4-yl]-(6-methyl-pyridin-2-ylmethyl)-amine,
[3-(4-Fluoro-phenyl)-thieno[2,3-b]pyridin-4-yl]-(6-methyl-pyridin-2-ylmethyl)-amine,
2-{3-Phenyl-4-[(pyridin-2-yhnethyl)-amino]-thieno[2,3-b]pyridin-6-yl}-propane-1,3-diol,
(4-Chloro-3-phenyl-thieno[2,3-b]pyridin-6-yl)-pyridin-2-ylmethyl-amine.

In one embodiment of the first aspect of the invention compounds of formula Ia are provided: 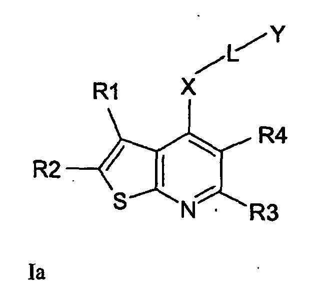 wherein:
R1 is C₆-C₁₀ aryl optionally substituted by cyano, halogen, nitro, trifluoromethyl, alkyl, alkylthio, alkoxy, NR9R10, CO₂R7, C(O)NR9R10, NC(O)R7, SO₂NR9R10 or hydroxyl , 5 to 10 membered heteroaryl, optionally substituted by cyano, nitro, halogen, alkyl, alkylthio, alkoxy, NR9R10, CO₂R7, C(O)NR9R10, NC(O)R7, SO₂NR9R10 or hydroxyl or C₃-C₆ cycloalkyl, optionally substituted by halogen, cyano, nitro, NR9R10, alkoxy, hydroxyl, unsubstituted heteroaryl, CO₂R7, C(O)NR9R10, NC(O)R7 or SO₂NR9R10;
R2 is H, alkyl, nitro, CO₂R7, CONR5R6 or halo;
R3 and R4 are H, NR5R6, NC(O)R7, halo, trifluromethyl, alkyl, CONR5R6, CO₂R7, nitrile or alkoxy;
R5 and R6 may be the same or different and may be H, alkyl, aryl, heteroaryl or cycloalkyl; or R5 and R6 may together form a saturated, unsaturated or partially saturated 4 to 7 member ring, wherein said ring may optionally comprise one or more further heteroatoms selected from N, O or S;
R7 is H or alkyl;
X is O, S or NR8;
R8 is H or alkyl;
L is (CH₂)ₙ, where n is 1, 2 or 3; and
Y is aryl, a heterocyclic group, alkyl, alkenyl or cycloalkyl;
Wherein R9 and R10 are selected from H, unsubstituted alkyl, unsubstituted aryl, unsubstituted heteroaryl, unsubstituted cycloalkyl, aminoethyl, methylaminoethyl, dimethylaminoethyl, hydroxyethyl, alkoxyethyl or R9 and R10 may together form a saturated, unsaturated or partially saturated 4 to 7 member ring;
or a pharmaceutically acceptable salt thereof.

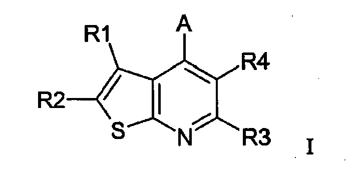

Compounds of formula I wherein A is X-L-Y and R3 is NR5R6, nitrile or alkoxy may be synthesised by reaction of compounds of formula II by displacement of the 6-chloro substituent with a suitable nucleophilic species. Such a reaction may be carried out with heating or microwave irradiation optionally in the presence of solvent and a base. 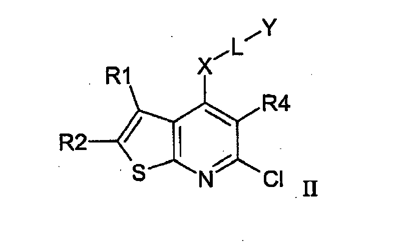 Compounds of formula II may be synthesized by reaction of compounds of formula III with a suitable nucleophile X-L-Y, where X, L and Y are as defined herein, optionally in the presence of a solvent and a base, and optionally at elevated temperature or with microwave irradiation. Preferably the solvent is N-methyl pyrrolidinone and the base is a hindered nitrogen base such as triethylamine. If a solvent is present the reaction is carried out at the reflux temperature of the solvent, or under sealed conditions and with microwave irradiation at a temperature of 120-200°C. Also isolable from this reaction is the product of substitution of the 6-chloro substituent.

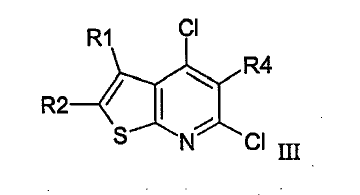

Compounds of formula III may be synthesized by reaction of a compound of formula IV with a chlorinating reagent such as phenylphosphonic dichloride or phosphorous oxychloride, optionally in the presence of a suitable solvent.

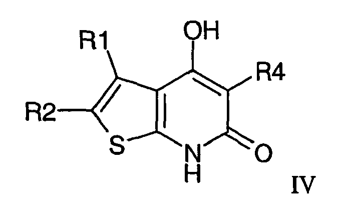

Compounds of formula IV where R4 is H may be synthesized from compounds of formula V by decarboxylation. This may be performed at elevated temperature, optionally in the presence of a solvent, optionally in the presence of an inorganic base, and optionally with microwave irradiation. If a solvent is present the reaction is carried out at the reflux temperature of the solvent, or under sealed conditions and with microwave irradiation at a temperature of 120-200°C. Preferably the solvent is water or ethanol or an admixture thereof and the base is an inorganic hydroxide preferably sodium or potassium hydroxide.

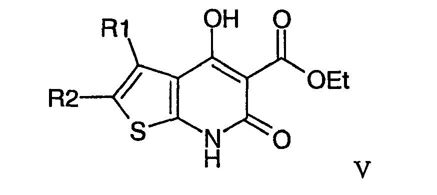

Compounds of formula V may be obtained by cyclisation of compounds of formula VI. This may be performed at elevated temperature, preferably in the presence of a solvent, preferably in the presence of an inorganic base, and optionally with microwave irradiation. If a solvent is present the reaction is carried out at the reflux temperature of the solvent, or under sealed conditions and with microwave irradiation at a temperature of 100-150°C. Preferably the solvent is tetrahydrofuran and the base is sodium hydride. 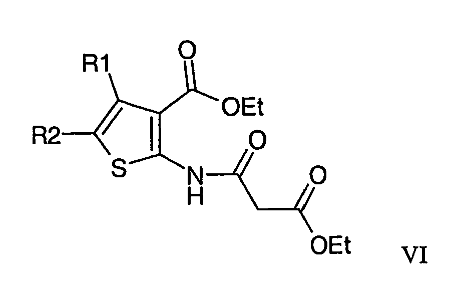 Compounds of formula VI may be synthesized from compounds of formula VII by reaction with diethyl malonate at elevated temperatures or, preferably, with ethyl malonyl chloride in a suitable solvent, preferably dichloromethane, and an organic nitrogen base, preferably triethylamine.

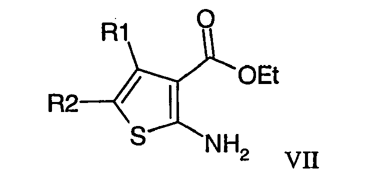

A compound of formula VII can be prepared by reaction of a compound of formula VIII with powdered sulphur, under basic conditions and in a suitable solvent. Preferably the base is triethylamine and the reaction is carried out at 25 to 65°C. The solvent may be an alcohol, preferably ethanol.

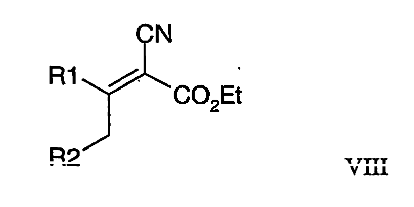

Compounds of formula VIII can be prepared by heating a compound of formula IX with ethylcyanoacetate (NCCH₂CO₂Et) in the presence of an acid and ammonium acetate in a suitable solvent, optionally with azeotropic water removal. Preferably the acid is acetic acid.

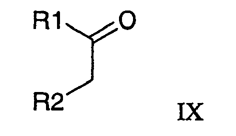

Compounds of formula IX are widely available from commercial sources or can be readily synthesised using standard synthetic organic chemistry procedures.

It is understood that compounds of formula I wherein R3 or R4 is an acid or ester group may undergo functional group transformation using methods familiar to those skilled in the art. In a preferred instance such compounds may undergo amidation by reaction with an alkyl or dialkylamine, or reduction with a reducing agent such as diisobutylaluminium hydride or lithium aluminium hydride.

In an alternative process, particularly applicable to those compounds of formula I wherein R1 is aryl, R2 is H, alkyl or halo, and R3 and R4 are H , a compound of formula X is reacted with a suitable nucleophile X-L-Y, where X, L and Y are as defined herein. Optionally the reaction may be carried out in the presence of a solvent and a base, and optionally at elevated temperature or with microwave irradiation. Preferably the solvent (if present) is an alcohol, preferably ethanol, and the base is a hindered nitrogen base such as triethylamine. If a solvent is present the reaction is carried out at the reflux temperature of the solvent, or under sealed conditions and with microwave irradiation at a temperature of 120-200°C.

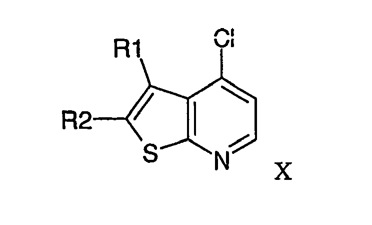

A compound of formula X may be obtained from a compound of formula XI by reaction with a chlorinating reagent such as phenylphosphonic dichloride or phosphorous oxychloride (or a mixture thereof) in a suitable solvent or no solvent, and with heating. Preferably the chlorinating reagent is phosphorous oxychloride and the reaction is carried out at reflux temperature and in the absence of additional solvent.

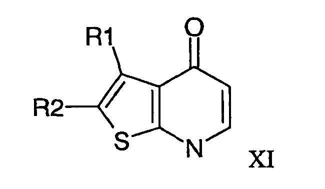

Compounds of formula XI may be obtained by the cyclisation of compounds of formula XII at elevated temperature, optionally in the presence of a solvent, and optionally with microwave irradiation. If a solvent is present the reaction is carried out at the reflux temperature of the solvent, or under sealed conditions and with microwave irradiation at a temperature of 120-200°C. Preferably the solvent is diphenyl ether or Dowtherm and the reaction is carried out at reflux temperature.

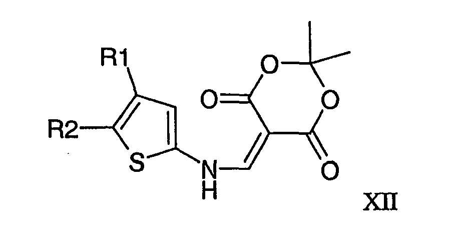

Compounds of formula XII may be obtained from compounds of formula XIII by reaction with Mander's reagent (the condensation product of 2,2-dimethyl-1,3-dioxane-4,6-dione, commonly known as Meldrum's acid, and triethyl orthoformate), at elevated temperature, optionally in the presence of a solvent at a temperature of 50-100°C. 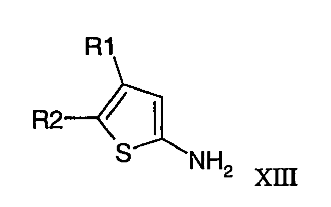 Compounds of formula XIII may be obtained from compounds of formula VII by decarboxylation. This may be performed at elevated temperature, optionally in the presence of a solvent, optionally in the presence of a base, and optionally with microwave irradiation. If a solvent is present the reaction is carried out at the reflux temperature of the solvent, or under sealed conditions and with microwave irradiation at a temperature of 120-200°C. Preferably the solvent is water or ethanol or an admixture thereof, and the base is an inorganic hydroxide, preferably sodium or potassium hydroxide.

In an alternative synthesis, suitable for compounds of formula I wherein R3 is H, dechlorination of compounds of formula II is carried out. Suitable conditions include the use of zinc powder in acetic acid at a temperature of 80-118°C. This process may also be applied to the side products of reaction of compounds of formula III with nucleophiles, wherein a compound substituted at the 6-position is formed and the remaining 4-chloro substituent removed to provide compounds of formula I wherein A is H.

Alternatively, compounds of formula I wherein R3 is a substituted alkyl group, in a preferred instance an acetic acid ester, can be prepared by the reaction of a compound of formula XIV, where the 4-position of the thienopyridine ring has a suitable leaving group W, by reaction with a suitable nucleophile X-L-Y, where X, L and Y are as defined herein, optionally in the presence of a solvent and a base, and optionally at elevated temperature or with microwave irradiation. Preferably the solvent (if present) is toluene, and the base is a hindered nitrogen base such as triethylamine. In a preferred instance the leaving group W is a halogen, preferably chlorine, or alternatively an alkyl or aryl sulfonate. In a more preferred instance the sulfonate is a trifluoromethanesulfonate. It is understood that compounds of formula I wherein R3 is an acetic acid ester may undergo transformations using methods familiar to those skilled in the art. In a preferred instance, compounds of formula I wherein R3 is a 1-hydroxyethyl group can be prepared by reaction of compounds of formula I wherein R3 is an acetic acid ester with a reducing agent such as diisobutylaluminium hydride or lithium aluminium hydride. In another preferred instance compounds of formula I wherein R3 is a 2-propane-1,3-diol may be obtained by alkylation of compounds of formula I wherein R3 is an acetic acid ester with a dialkyl carbonate or a chloroformate, and reduction of the 1,3-diester formed thereby with a reducing agent such as diisobutylaluminium hydride or lithium aluminium hydride.

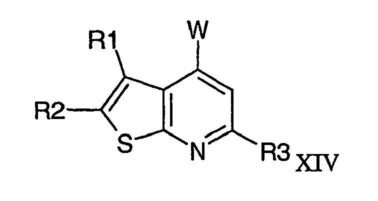

Compounds of formula XIV wherein W is an alkyl or aryl sulfonate can be obtained from a compound of formula XV by reaction with sulfonating agent, such as a sulfonyl chloride or sulfonic anhydride, in a preferred instance trifluoromethanesulfonic anhydride, in the presence of a solvent and a base, and optionally at elevated temperature or with microwave irradiation. Preferably the solvent is dichloromethane, and the base is a nitrogen base such as pyridine. Compounds of formula XIV wherein W is a halogen, in a preferred instance chlorine, can be obtained from a compound of formula XV by reaction with a chlorinating reagent such as phenylphosphonic dichloride or phosphorous oxychloride (or a mixture thereof) in a suitable solvent or no solvent, and with heating. Preferably the chlorinating reagent is phosphorous oxychloride and the reaction is carried out at reflux temperature and in the absence of additional solvent.

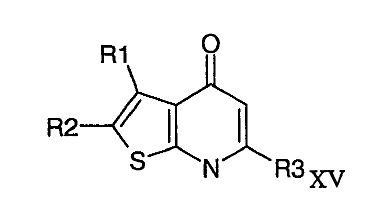

Compounds of formula XV may be obtained from a compound of formula XVI by an intramolecular cyclisation at elevated temperatures. The reaction may involve Lewis acid catalysis such as aluminium trichloride, or mineral acid catalysis such as polyphosphoric acid, optionally in the presence of solvent or as a melt. In a more preferred instance the cyclisation may be induced thermally by heating in a suitable high-boiling solvent, optionally in a microwave. A preferred solvent is diphenyl ether and the reaction is carried out at the reflux temperature of the solvent.

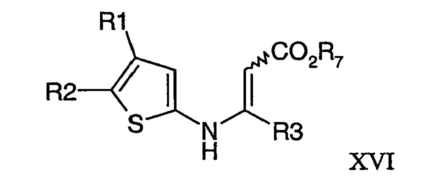

Compounds of formula XVI may be obtained from compounds of formula XIII by enamine formation with suitably substituted ketones, in a preferred instance diethylacetone dicarboxylate. This reaction may be carried out in the presence of solvent under acid catalysis with removal of water by azeotropic distillation or molecular sieves.

Compounds of formula I wherein A is a halogen group, in particular a chloride substituent, can be isolated as minor products when a compound of formula III is reacted with a nucleophile X-L-Y. It will be understood by those skilled in the art that further manipulation of the chloride substituent in this instance allows the synthesis of those compounds wherein A is hydrogen.

Many of the starting materials referred to in the reactions described above are available from commercial sources or can be made by methods cited in the literature references. Synthetic methods for thienopyridines may be found in references such as Gewald *et al* (1979), Munchof *et al* (2004), Barker *et al* (1985), Charvát *et al* (1995) and articles cited therein. Synthetic methods can also be found in reviews; thiophenes for example can be found in references cited in Comprehensive Heterocyclic Chemistry, Eds Katritzky, A. R., Rees, C. R., (4), 863-934, and Comprehensive Heterocyclic Chemistry (II), Eds Katritzky, A. R., Rees, C. W., Scriven, E. F. V., (2). 607-678.

Suitable starting materials include:

| Material | Reference | Supplier |
|---|---|---|
| Ethyl Malonyl Chloride | 16,387-2 | Aldrich |
| 4-Fluoroacetophenone | F-320-7 | Aldrich |
| Acetophenone | A1 070-1 | Aldrich |
| 2-(Aminomethyl)pyridine | A6,520-4 | Aldrich |
| Diethanolamine | D8,330-3 | Aldrich |
| ethanolamine | 41,100-0 | Aldrich |
| Propiophenone | P5,160-5 | Aldrich |
| Benzylamine | B1,630-5 | Aldrich |
| 2, 2-Dimethyl-1,3-dioxane-4,6-dione | 21,014-5 | Aldrich |
| Triethyl orthoformate | 30,405-0 | Aldrich |
| Diethyl-1,3-Acetone Dicarboxylate | 16,512-3 | Aldrich |

As discussed herein, the compounds of the invention are useful in the treatment of various conditions. Thus, in a second aspect, the present invention provides a compound of formula I or Ia as defined herein for use in medicine. In a further, aspect the present invention provides a pharmaceutical formulation comprising at least one compound of formula I or Ia as defined herein and optionally one or more excipients, carriers or diluents.

The compositions of the invention may be presented in unit dose forms containing a predetermined amount of each active ingredient per dose. Such a unit may be adapted to provide 5-100mg/day of the compound, preferably either 5-15mg/day, 10-30mg/day, 25-50mg/day 40-80mg/day or 60-100mg/day. For compounds of formula I, doses in the range 100-1000mg/day are provided, preferably either 100-400mg/day, 300-600mg/day or 500-1000mg/day. Such doses can be provided in a single dose or as a number of discrete doses. The ultimate dose will depend on the condition being treated, the route of administration and the age, weight and condition of the patient and will be at the doctor's discretion.

The compositions of the invention may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier(s) or excipient(s).

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

Pharmaceutical formulations adapted for transdermal administration may be presented as discrete patches intended to remain in intimate contact with the epidermis of the recipient for a prolonged period of time. For example, the active ingredient may he delivered from the patch by iontophoresis as generally described in Pharmaceutical Research, 3(6), 318 (1986).

Pharmaceutical formulations adapted for topical administration may be formulated as ointments, creams, suspensions, lotions, powders, solutions, pastes, gels, sprays, aerosols or oils.

For applications to the eye or other external tissues, for example the mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical formulations adapted for topical administration to the eye include eye drops wherein the active ingredient is dissolved or suspended in a suitable carrier, especially an aqueous solvent.

Pharmaceutical formulations adapted for topical administration in the mouth include lozenges, pastilles and mouth washes.

Pharmaceutical formulations adapted for rectal administration may be presented as suppositories or enemas.

Pharmaceutical formulations adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical formulations adapted for administration by inhalation include fine particle dusts or mists which may be generated by means of various types of metered dose pressurised aerosols, nebulizers or insufflators.

Pharmaceutical formulations adapted for vaginal administration may be presented as pessaries, tampons, creams, gels, pastes, foams or spray formulations.

Pharmaceutical formulations adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

Preferred unit dosage formulations are those containing a daily dose or sub-dose, as herein above recited, or an appropriate fraction thereof, of an active ingredient.

It should be understood that in addition to the ingredients particularly mentioned above, the formulations may also include other agents conventional in the art having regard to the type of formulation in question, for example those suitable for oral administration may include flavouring agents.

The compounds or compositions of the invention can be used to treat conditions which require inhibition of potassium channels, for example in the treatment of arrythmia. Thus, in a further aspect, the present invention provides:

### (ii) the use of a compound of the invention in the manufacture of a medicament for use in potassium channel inhibition.

In particular, the medicament is for use in the treatment or prevention of arrhythmia, type-2 diabetes and immunological disorders including rheumatoid arthritis, type-1 diabetes, inflammatory bowel disorder and demyelinating disorders such as multiple sclerosis.

### Examples

Using the information outlined herein the following compounds can be synthesised which are given by way of example only. The pharmacological profile of compounds of the present invention can readily be assessed by those skilled in the art using routine experimentation, such as procedures and techniques illustrated herein and described in detail in Ford *et al.,* 2002.

### Example 1

### 2-Cyano-3-phenyl-but-2-enoic acid ethyl ester

A stirred mixture of acetophenone (180g, 1.5mol), ethyl cyanoacetate (170g, 1.3mol), ammonium acetate (23.1g), acetic acid (72g) and toluene (300ml) was heated under reflux for 18 hours while water was removed from the reaction by azeotropic distillation. The mixture was allowed to cool to ambient temperature, toluene (100ml) was added, then the mixture was washed with water (3 x 100ml). The combined aqueous washings were shaken with toluene (50ml), then the combined toluene solutions were dried over magnesium sulphate, filtered and the solvent was removed *in vacuo*. The residual oil was distilled under reduced pressure to give 2-cyano-3-phenyl-but-2-enoic acid ethyl ester as an oil which was used without further purification.

### Examples 2 and 3

The compounds set out below were prepared in the same way as in Example 1, using the appropriate starting materials.

| Example | Compound |
|---|---|
| 2 | 2-Cyano-3-(4-fluoro-phenyl)-but-2-enoic acid ethyl ester |
| 3 | 2-Cyano-3-phenyl-pent-2-enoic acid ethyl ester |

### Example 4

### 2-Amino-4-phenyl-thiophene-3-carboxylic acid ethyl ester

2-Cyano-3-phenyl-but-2-enoic acid ethyl ester (513.25g, 2.3mol) was added at ambient temperature to a vigorously-stirred suspension of powdered sulfur (76g, 2.3mol) in ethanol (500ml). Diethylamine (200ml) was added in portions over 20 minutes, during which time the temperature of the reaction rose to 62°C. The mixture was allowed to cool to 36°C, then it was heated to 50°C and stirring at that temperature was continued for 1hr. After this time, stirring was discontinued, the hot solution was removed by decantation from unreacted sulfur, then it was allowed to cool to ambient temperature. The resulting solid was collected by filtration, washed with a little cold ethanol and dried *in vacuo* to give 2-amino-4-phenylthiophene-3-carboxylic acid ethyl ester as an orange solid which was used without further purification.

### Examples 5 and 6

The compounds set out below were prepared in the same way as in Example 4, using the appropriate starting materials.

| Example | Compound |
|---|---|
| 5 | 2-Amino-4-(4-fluoro-phenyl)-thiophene-3-carboxylic acid ethyl ester |
| 6 | 2-Amino-5-methyl-4-phenyl-thiophene-3-carboxylic acid ethyl ester |

### Example 7

### 2-(2-Ethoxycarbonyl-acetylamino)-4-phenyl-thiophene-3-carboxylic acid ethyl ester

2-Amino-4-phenyl-thiophene-3-carboxylic acid ethyl ester (5.0g, 0.02M) was dissolved in anhydrous dichloromethane (150ml). Triethylamine (5.56ml, 0.04M) was added and the mixture cooled to 0°C. Ethyl Malonyl Chloride (3.79ml, 0.03M) was added over 5 min maintaining the temperature at 0°C. The reaction was then stirred at room temperature for 1hr. Water (100ml) was added and the organic layer separated. The aqueous layer was extracted with a further 100ml of dichloromethane. The organics were combined, washed with water (2 x 100ml) and dried over sodium sulphate. The concentrated residues were columned on silica, eluting with ethylacetate-cyclohexane 5-10% v/v. Pure fractions were combined and concentrated and the residues triturated with hexane, decanted and dried to give 2-(2-ethoxycarbonyl-acetylamino)-4-phenyl-thiophene-3-carboxylic acid ethyl ester as a white solid. Yield = 6.95g (96.2%).

### Examples 8 and 9

The compounds set out below were prepared in the same way as in Example 7, using the appropriate starting materials.

| Example | Compound |
|---|---|
| 8 | 2-(2-Ethoxycarbonyl-acetylamino)-4-(4-fluoro-phenyl)-thiophene-3-carboxylic acid ethyl ester |
| 9 | 2-(2-Ethoxycarbonyl-acetylamino)-5-methyl-4-phenyl-thiophene-3-carboxylic acid ethyl ester |

### Example 10

### 4-Hydroxy-6-oxo-3-phenyl-6,7-dlhydro-thieno[2,3-b]pyridine-5-carboxylic acid ethyl ester

2-(2-Ethoxycarbonyl-acetylamino)-4-phenyl-thiophene-3-carboxylic acid ethyl ester (5.0g, 13.8mmol) and sodium hydride (1.1g, 27.7mmol) in anhydrous THF (120 ml) were refluxed for 6hr under nitrogen. On cooling, solvents were removed in vacuo and the residue suspended in water (100ml). The mixture was acidified by addition of concentrated hydrochloric acid (5ml) and stirred for 1hr. The precipitate was filtered and dried, then recrystallised from ethanol to give 4-hydroxy-6-oxo-3-phenyl-6,7-dihydro-thieno[2,3-b]pyridine-5-carboxylic acid ethyl ester as a pale yellow solid. Yield = 2.59g (63.3%).

### Examples 11 and 12

The compounds set out below were prepared in the same way as in Example 10, using the appropriate starting materials.

| Example | Compound |
|---|---|
| 11 | 3-(4-Fluoro-phenyl)-4-hydroxy-6-oxo-6,7-dihydro-thieno[2,3-b]pyridine-5-carboxylic acid ethyl ester |
| 12 | 4-Hydroxy-2-methyl-6-oxo-3-phenyl-6,7-dihydro-thieno[2,3-b]pyridine-5-carboxylic acid ethyl ester |

### Example 13

### 4-Hydroxy-3-phenyl-7H-thieno[2,3-b]pyridin-6-one

4-Hydroxy-6-oxo-3-phenyl-6,7-dihydro-thieno[2,3-b]pyridine-5-carboxylic acid ethyl ester (1.5g, 4.76mmol) was refluxed in 2M sodium hydrxide (50ml) for 5hr, filtered while still hot, then cooled to 0°C and acidified to pH 1 by addition of conc. HCl. The resultant white precipitate was filtered and dried to give 4-hydroxy-3-phenyl-7H-thieno[2,3-b]pyridin-6-one as a white solid. Yield = 1.05g (90.7%).

### Examples 14 and 15

The compounds set out below were prepared in the same way as in Example 13, using the appropriate starting materials.

| Example | Compound |
|---|---|
| 14 | 3-(4-Fluoro-phenyl)-4-hydroxy-7H-thieno[2,3-b]pyridin-6-one |
| 15 | 14-Hydroxy-2-methyl-3-phenyl-7H-thieno[2,3-b]pyridin-6-one |

### Example 16

### 4,6-Dichloro-3-phenyl-thieno[2,3-b]pyridine

A mixture of 4-hydroxy-3-phenyl-7H-thieno[2,3-b]pyridin-6-one (1.05g, 4.32mmol) in phenyl phosphonic dichloride (20ml) was heated to 180°C for 3hr. On cooling, the reaction was poured into ice and stirred for 30min. The aqueous was extracted with DCM (3 x 100ml). The extracts were combined, washed with water, dried over sodium sulphate and concentrated. The residue was columned on silica to give 4,6-dichloro-3-phenyl-thieno[2,3-b]pyridine as an opaque oil which slowly crystallised to a pale yellow solid. Yield = 0.505 g (42.9 %).

### Examples 17 and 18

The compounds set out below were prepared in the same way as in Example 16, using the appropriate starting materials.

| Example | Compound |
|---|---|
| 17 | 4,6-Dichloro-3-(4-fluoro-phenthieno[2,3-b]pyridine |
| 18 | 4,6-Dichloro-2-methyl-3-phenyl-thieno[2,3-b]pyridine |

### Examples 19 and 20

### (6-Chloro-3-phenyl-thieno[2,3-b]pyridin-4-yl)-pyridin-2-ylmethyl-amine

A mixture of 4,6-dichloro-3-phenyl-thieno[2,3-b]pyridine (400mg, 1.43mmol) and 2-aminomethylpyridine (294µl, 1.86mmol) in NMP (1ml) were heated in the microwave at 200 °C for 1 h. The reaction was diluted with water (30 ml) and DCM (30 ml). The DCM layer was separated and washed with water (6 x 50 ml), dried (Na₂SO₄), and concentrated. The residue was columned on silica, eluting (EtOAc/Hexane 0 - 100%). The first isolated fraction gave (6-chloro-3-phenyl-thieno[2,3-b]pyridin-4-yl)-pyridin-2-ylmethyl-amine (19) as colourless crystals. Yield = 288 mg (57.3%). The second isolated fraction gave (4-Chloro-3-phenyl-thieno[2,3-b]pyridin-6-yl)-pyridin-2-ylmethyl-amine (20) as an orange oil. Yield = 24mg (4.7%).

### Examples 21 to 23

The compounds set out below were prepared in the same way as in Example 19, using the appropriate starting materials.

| Example | Compound |
|---|---|
| 21 | [6-Chloro-3-(4-fluoro-phenyl)-thieno[2,3-b]pyridin-4-yl]-pyidin-2-ylmethyl-amine |
| 22 | [6-Chloro-3-(4-fluoro-phenyl)-thieno[2,3-b]pyridin-4-yl]-(6-methyl-pyridin-2-ylmethyl)-amine |
| 23 | (6-Chloro-2-methyl-3-phenyl-thieno[2,3-b]pyridin-4-yl)-pyridin-2-ylmethyl-amine |

### Example 24

### 2-{3-Phenyl-4-[(pyridin-2-ylmethyl)-amino]-thieno[2,3-b]pyridin-6-ylamino}-ethanol

A mixture of (6-chloro-3-phenyl-thieno[2,3-b]pyridin-4-yl)-pyridin-2-ylmethyl-amine (20mg, 0.57mmol) and ethanolamine (1ml) were heated to 200°C in the microwave and maintained at this temperature for 90min. On cooling, the reaction mixture was poured into DCM (50ml) and washed with water (2 x 50ml), dried (Na₂SO₄), and concentrated. The residue was purified on silica (ethyl acetate, 100%) to give 2-{3-phenyl-4-[(pyridin-2-ylmethyl)-amino]-thieno[2,3-b]pyridin-6-ylamino}-ethanol as a white solid. Yield = 18 mg (83.9 %).

### Examples 25 to 30

The compounds set out below were prepared in the same way as in Example 24, using the appropriate starting materials.

| Example | Compound |
|---|---|
| 25 | 2-((2-Hydroxy-ethyl)-{3-phenyl-4-[(pyridin-2-ylmethyl)-amino]-thieno[2,3-b]pyridin-6-yl}-amino)-ethanol |
| 26 | 2-[{3-(4-Fluoro-phenyl)-4-[(pyridin-2-ylmethyl)-amino]-thieno[2,3-b]pyridin-6-yl}-(2-hydroxy-ethyl)-amino]-ethanol |
| 27 | 2-{3-(4-Fluoro-phenyl)-4-[(pyridin-2-ylmethyl)-amino]-thieno[2,3-b]pyridin-6-ylamino}-ethanol |
| 28 | 2-[{3-(4-Fluoro-phenyl)-4-[(6-methyl-pyridin-2-ylmethyl)-amino]-thieno[2,3-b]pyridin-6-yl}-(2-hydroxy-ethyl)-amino]-ethanol |
| 29 | 2-{3-(4-Fluoro-phenyl)-4-[(6-methyl-pyridin-2-ylmethyl)-amino]-thieno[2,3-b]pyridin-6-ylamino}-ethanol |
| 30 | 2-{2-Methyl-3-phenyl-4-[(pyridin-2-ylmethyl)-amino]-thieno[2,3-b]pyridin-6-ylamino}-ethanol |

### Example 31

### 4-Phenyl-thiophen-2-ylamine

2-Amino-4-phenyl-thiophene-3-carboxylic acid ethyl ester (5g, 20.2mmol) was suspended in 20% potassium hydroxide solution (100ml) and heated to reflux. Ethanol (100ml) was added to aid dissolution. The reaction was stirred overnight and then cooled to room temperature and diluted with water (250ml). The precipitated solid was collected by filtration and dissolved in DCM/ethyl acetate before drying over magnesium sulfate. The solvent was removed in vacuo to give 4-phenyl-thiophen-2-ylamine. Yield 1.05g (30%).

### Example 32

### 2,2-Dimethyl-5-[(4-phenyl-thiophen-2-ylamino)-methylene]-[1,3]dioxane-4,6-dione

Meldrum's acid (1.05g, 7.3mmol) was added to triethyl orthoformate (50ml) and stirred at 30°C for 1hr. The reaction mixture was cooled to room temperature and 4-phenyl-thiophene-2-yl-amine (1.07g, 6.1mmol) was added in portions. The reaction mixture was heated to 85°C and stirred at this temperature overnight before cooling to room temperature. The solvent was removed in vacuo to give a residue which was dissolved in DCM and treated with potassium carbonate. After stirring for 30 min the mixture was filtered and the solvent removed in vacuo to give 2,2-dimethyl-5-[(4-phenyl-thiophen-2-ylamino)-methylene]-[1,3]dioxane-4,6-dione. Yield = 1.41g (70%).

### Example 33

### 3-Phenyl-7H-thieno[2,3-b]pyridine-4-one

Diphenyl Ether (15ml) was heated to reflux and 2,2-dimethyl-5-[(4-phenyl-thiophen-2-ylamino)-methylene]-[1,3]dioxane-4,6-dione (1.41g, 4.29mmol) was added in portions with evolution of gas. The reaction mixture was kept at reflux for a further 45 min, before cooling to room temperature. Trituration with diisopropyl ether and petroleum ether (40-60°) gave 3-phenyl-7H-thieno[2,3-b]pyridine-4-one. Yield = 0.7g, (72%).

### Example 34

### 4-Chloro-3-phenyl-thieno[2,3-b]pyridine

3-Phenyl-7H-thieno[2,3-b]pyridine-4-one (0.7g, 4.29mmol) was added to phosphorous oxychloride (10ml) and heated to reflux for 4hr. The solvent was removed to near dryness and the residue dissolved in DCM. The solution was washed with water followed by saturated sodium hydrogen carbonate and dried over magnesium sulfate. The solution was filtered through a pad of silica and solvents removed in vacuo to give 4-chloro-3-phenyl-thieno[2,3-b]pyridine. Yield = 0.261g (25%).

### Example 35

### (3-Phenyl-thieno[2,3-b]pyridin-4-yl)-pyridin-2-ylmethyl-amine

4-Chloro-3-phenyl-thieno[2,3-b]pyridine (47mg, 0.19mmol) and 2-aminomethyl pyridine (0.5ml, 4.85mmol) were placed in a 10ml glass tube. The vessel was sealed with a septum and placed in the microwave cavity. Using microwave irradiation the temperature was ramped from room temperature to 150°C. Once 150°C was reached, the reaction mixture was held at this temperature for 90 minutes. After cooling to room temperature, the temperature was ramped to 200°C and held at this temperature for 30min. After cooling, the reaction mixture was diluted with DCM and washed with water. The organic phase was dried over magnesium sulfate, filtered and concentrated in vacuo. The residue was purified by preparative HPLC to give (3-phenyl-thieno[2,3-b]pyridin-4-yl)-pyridin-2-ylmethyl-amine. Yield = 14 mg, 23%.

### Example 36

### [3-(4-Fluoro-phenyl)-thieno[2,3-b]pyridin-4-yl]-pyridin-2-ylmethyl-amine

[6-Chloro-3-(4-fluoro-phenyl)-thieno[2,3-b]pyridin-4-yl]-pyridin-2-ylmethyl-amine (40 mg, 0.1mmol) was dissolved in acetic acid (3ml). Zinc powder (71mg, 1mmol) was added and the mixture refluxed for 6hr. On cooling, the mixture was filtered through celite and the solids washed with further portions of acetic acid (3 x 5 ml). The acetic acid extracts were neutralized with saturated sodium bicarbonate solution and extracted with DCM (2 x 50ml). The extracts were combined, dried over sodium sulfate and concentrated. The residue was purified on silica (ethyl actetate/hexane 20 - 40%) to give [3-(4-fluoro-phenyl)-thieno[2,3-b]pyridin-4-yl]-pyridin-2-ylmethyl-amine as a white solid. Yield = 5.5mg (15.2 %).

### Example 37

The compound set out below was prepared in the same way as in Example 36, using the appropriate starting materials.

| Examples | Compound |
|---|---|
| 37 | [3-(4-Fluoro-phenyl)-thieno[2,3-b]pyridin-4-yl]-(6-methyl-pyridin-2-ylmethyl)-amine |

### Example 38

### (E/Z)-3-(4-Phenyl-thiophen-2-ylamino)-pent-2-enedioic acid diethyl ester

Amino thiophene (1.39g, 7.94mmol), p-Toluenesulfonic acid (7.5mg, 0.4mmol) and diethylacetonedicarboxylate (1.73ml), 9.53mmol) were refluxed for 12h in chloroform in the presence of 3Å molecular sieves. On cooling, the reaction was filtered, treated with activated charcoal, refiltered through a pad of celite and concentrated. The residue was triturated with petroleum ether (bp.40-60°) until crystallisation was complete to give (E/Z)-3-(4-Phenyl-thiophen-2-ylamino)-pent-2-enedioic acid diethyl ester as a brown powder. Yield = 2.4g (84%, mixture of E and Z isomers).

### Example 39

### (4-Oxo-3-phenyl-4,7-dihydro-thieno[2,3-b]pyridin-6-yl)-acetic acid ethyl ester

(E/Z)-3-(4-Phenyl-thiophen-2-ylamino)-pent-2-enedioic acid diethyl ester was added portionwise to refluxing Diphenyl ether (20ml). Once addition was complete, the mixture was refluxed for a further 30min. On cooling, the reaction was diluted with (bp.40-60°) petroleum ether (100ml) and stirred vigorously for 1h. The initially formed red gum slowly solidified to a fine yellow precipitate. This was filtered, and washed with boiling (bp.40-60°) petroleum ether (2x50ml) and dried under vacuum to give (4-Oxo-3-phenyl-4,7-dihydro-thieno[2,3-b]pyridin-6-yl)-acetic acid ethyl ester as a yellow powder. Yield = 1.89g, (71 %).

### Example 40

### (3-Phenyl-4-trifluoromethanesulfonyloxy-thieno[2,3-b]pyridin-6-yl)-acetic acid ethyl ester

(4-Oxo-3-phenyl-4,7-dihydro-thieno[2,3-b]pyridin-6-yl)-acetic acid ethyl ester (0.8g, 2.5mmol), and pyridine (0.2ml, 2.5mmol) were dissolved in DCM (5ml) under nitrogen and cooled to 0°C. Trifluoromethanesulfonic anhydride (0.42ml, 2.5mmol) was added dropwise and the reaction stirred for 18h at room temperature. Solvents were removed in vacuo, and the residue was diluted with water (100ml) and extracted with DCM (2x50ml). The extracts were combined, dried over magnesium sulphate, filtered and concentrated to a yellow oil, which solidified on prolonged standing to a waxy yellow solid. This was purified on silica, eluting (DCM/ petroleum ether (bp.40-60°)), 0 - 50%) to give (3-Phenyl-4-trifluoromethanesulfonyloxy-thieno[2,3-b]pyridin-6-yl)-acetic acid ethyl ester as a yellow solid. Yield = 878mg, (78%).

### Example 41

### {3-Phenyl-4-[(pyridin-2-ylmethyl)-amino]-thieno[2,3-b]pyridin-6-yl}-acetic acid ethyl ester

Thienotriflate (Example 40, 0.9g, 2mmol), and Hünigs base (0.7ml, 4mmol) were dissolved in dry toluene (20ml) under nitrogen. 2-Aminomethylpyridine (0.2ml, 2mmol) was added and the mixture refluxed for 72h. On cooling, the reaction was washed with water (2x50ml), dried over magnesium sulfate, filtered and concentrated. The residue was purified on silica, eluting (Ethyl Acetate/petroleum ether (bp.40-60°)), 0-50%) to give {3-Phenyl-4-[(pyridin-2-ylmethyl)-amino]-thieno[2,3-b]pyridin-6-yl}-acetic acid ethyl ester as a yellow solid. Yield = 186mg (26%).

### Example 42

### 2-{3-Phenyl-4-[(pyridin-2-ylmethyl)-amino]-thieno[2,3-b]pyridin-6-yl}-malonic acid diethyl ester

{3-Phenyl-4-[(pyridin-2-ylmethyl)-amino]-thieno[2,3-b]pyridin-6-yl}-acetic acid ethyl ester (180mg, 0.45mmol) was dissolved in dry THF (10ml) under nitrogen. Diethyl Carbonate (0.27ml, 2.23mmol) was added and the mixture cooled to 0°C. Sodium Hydride (36mg, 0.89mmol) was added and the mixture stirred at 0°C for a further 10min, then at room temperature for 20min, then brought to reflux for 45min. On cooling, the reaction was diluted with water (100ml) and extracted with DCM (3x50ml), the extracts combined, dried over magnesium sulphate and concentrated. The residue was purified on silica, eluting (Ethyl Acctatc/DCM), 0-20% to give 2{3-Phenyl-4-[(pyridin-2-ylmethyl)-amino]-thieno[2,3-b]pyridin-6-yl}-malonic acid diethyl ester as a yellow solid. Yield = 96mg (45%).

### Example 43

### 2-{3-Phenyl-4-[(pyridin-2-ylmethyl)-amino]-thieno[2,3-b]pyridin-6-yl}-ethanol

{3-Phenyl-4-[(pyridin-2-ylmethyl)-amino]-thieno[2,3-b]pyridin-6-yl}-acetic acid ethyl ester (47mg, 0.12mmol) was dissolved in dry THF (10ml) under nitrogen and cooled to 0°C. Diisobutyl Aluminium Hydride (0.466ml of a 1M solution in hexanes, 0.466mmol) was added dropwise over 2min and the mixture stirred at 0°C for 1h, then stirred at room temperature overnight. The reaction was cooled to 0°C and water (5ml) was added carefully, followed by 1M Rochelle's salt (5ml). The reaction was stirred at room temperature for 15min, and then extracted with DCM (2x25ml), the extracts combined, dried over magnesium sulphate, and concentrated. The residue was purified on silica, eluting (Ethyl Acetate/Petroleum ether), 50-100%) to give 2-{3-Phenyl-4-[(pyridin-2-ylmethyl)-amino]-thieno[2,3-b]pyridin-6-yl}-ethanol as a brown solid. Yield = 25.3mg (60.4%).

### Example 44

### 2-{3-Phenyl-4-[(pyridin-2-ylmethyl)-amino]-thieno[2,3-b]pyridin-6-yl}-propane-1,3-diol

A solution of 2-{3-Phenyl-4-[(pyridin-2-ylmethyl)-amino]-thieno[2,3-b]pyridin-6-yl}-malonic acid diethyl ester (87mg, 0.18mmol) in dry THF (10ml) was cooled to 0°C under nitrogen. Diisobutyl Aluminium Hydride (1.46ml of a 1M solution in hexanes, 1.46mmol) was added dropwise over 2min and the mixture stirred at 0°C for 1h, then allowed to reach room temperature overnight. The reaction was quenched at 0°C by adition of 1M Rochelle's salt (10ml). The mixture was extracted with DCM (3x20ml), the extracts combined, washed with brine (50ml), dried over magnesium sulphate and concentrated. The residue was purified on silica, eluting (Ethyl Acetate, 100%) to give 2-{3-Phenyl-4-[(pyridin-2-ylmethyl)-amino]-thieno[2,3-b]pyridin-6-yl}-propane-1,3-diol as a yellow oil. Yield = 22.5mg, (31.4%).

### Example 45

### Analytical Data for compounds representative of the above examples are shown in the table below.

| Example | Compound Name | Mass Spectrum (m/z) |
|---|---|---|
| 7 | 2-(2-Ethoxycarbonyl-acetylamino)-4-phenyl-thiophene-3-carboxylic acid ethyl ester | 7.84 min, 360 (ES-, 100%, [M-H]) |
| 8 | 2-(2-Ethoxycarbonyl-acetylamino)-4-(4-fluoro-phenyl)-thiophene-3-carboxylic acid ethyl ester | 7.80 min, 378 (ES-, 100%, [M-H]) |
| 9 | 2-(2-Ethoxycarbonyl-acetylamino)-5-methyl-4-phenyl-thiophene-3-carboxylic acid ethyl ester | 8.06 min, 374, (ES+, 100%, [M+H]+) |
| 10 | 4-Hydroxy-6-oxo-3-phenyl-6,7-dihydro-thieno[2,3-b]pyridine-5-carboxylic acid ethyl ester | 6.50 min, 316 (ES+, 100%, [M+H]) |
| 11 | 3-(4-Fluoro-phenyl)-4-hydroxy-6-oxo-6,7-dihydro-thieno[2,3-b]pyridine-5-carboxylic acid ethyl ester | 6.51 min, 334 (ES+, 100%, [M+H]) |
| 12 | 4-Hydroxy-2-methyl-6-oxo-3-phenyl-6,7-dihydro-thieno[2,3-b]pyridine-5-carboxylic acid ethyl ester | 6.86 min, 330 (ES+, 100%, [M+H]) |
| 13 | 4-Hydroxy-3-phenyl-7H-thieno[2,3-b]pyridin-6-one | 5.71 min, 244 (ES+, 100%, [M+H]) |
| 14 | 3-(4-Fluoro-phenyl)-4-hydroxy-7H-thieno[2,3-b]pyridin-6-one | 5.75 min, 262 (ES+, 100%, [M+H]) |
| 15 | 4-Hydroxy-2-methyl-3-phenyl-7H-thieno[2,3-b]pyridin-6-one | 5.92 min, 258 (ES+, 100%, [M+H]) |
| 19 | (6-Chloro-3-phenyl-thieno[2,3-b]pyridin-4-yl)-pyridin-2-ylmethyl-amine | 8.00 min, 352 (ES+, 100%, [M+H]) |
| 21 | [6-Chloro-3-(4-fluoro-phenyl)-thieno[2,3-b]pyridin-4-yl]-pyridin-2-ylmethyl-amine | 8.16 min, 370, (ES+, 100%, [M+H]) |
| 22 | [6-Chloro-3-(4-fluoro-phenyl)-thieno[2,3-b]pyridin-4-yl]-(6-methyl-pyridin-2-ylmethyl)-amine | 8.34 min, 384, (ES+, 100%, [M+H]) |
| 23 | (6-Chloro-2-methyl-3-phenyl-thieno[2,3-b]pyridin-4-yl)-pyridin-2-ylmethyl-amine | 8.17 min, 366 (ES+, 100%, [M+H]) |
| 24 | 2-{3-Phenyl-4-[(pyridin-2-ylmethyl)-amino]-thieno[2,3-b]pyridin-6-ylamino}-ethanol | 6.50 min, 377, (ES+, 100%, [M+H]) |
| 25 | 2-((2-Hydroxy-ethyl)-{3-phenyl-4-[(pyridin-2-ylmethyl)-amino]-thieno[2,3-b]pyridin-6-yl} -amino)-ethanol | 6.45 min, 421, (ES+ 100%, [M+H]) |
| 26 | 2-[{3-(4-Fluoro-phenyl)-4-[(pyridin-2-ylmethyl)-amino]-thieno[2,3-b]pyridin-6-yl}-(2-hydroxy-ethyl)-amino]-ethanol | 6.76 min, 439, (ES+ 100%, [M+H]) |
| 27 | 2-{3-(4-Fluoro-phenyl)-4-[(pyridin-2-ylmethyl)-amino]-thieno[2,3-b]pyridin-6-ylamino}-ethanol | 6.75 min, 395, (ES+, 100%, [M+H]) |
| 28 | 2-[{3-(4-Fluoro-phenyl)-4-[(6-methyl-pyridin-2-ylmethyl)-amino]-thieno[2,3-b]pyridin-6-yl}-(2-hydroxy-ethyl)-amino]-ethanol | 6.87 min, 453, (ES+, 100%, [M+H]) |
| 29 | 2-{3-(4-Fluoro-phenyl)-4-[(6-methyl-pyridin-2-ylmethyl)-amino]-thieno[2,3-b]pyridin-6-ylamino}-ethanol | 6.70 min, 409, (ES+, 100%, [M+H]) |
| 30 | 2-{2-Methyl-3-phenyl-4-[(pyridin-2-ylmethyl)-amino]-thieno[2,3-b]pyridin-6-ylamino}-ethanol | 6.83, 391 (ES+, 100%, [M+H]) |
| 36 | [3-(4-Fluoro-phenyl)-thieno[2,3-b]pyridin-4-yl]-pyridin-2-ylmethyl-amine | 7.28 min, 336 (ES+, 100%, [M+H]) |
| 37 | [3-(4-Fluoro-phenyl)-thieno[2,3-b]pyridin-4-yl]-(6-methyl-pyridin-2-ylmethyl)-amine | 7.25 min, 350 (ES+, 100%, [M+H]) |
| 40 | (3-Phenyl-4-trifluoromethanesulfonyloxy-thieno[2,3-b]pyridin-6-yl)-acetic acid ethyl ester | 7.96 min, 446 (ES+, 100%, [M+H]) |
| 41 | {3-Phenyl-4-[(pyridin-2-ylmethyl)-amino]-thieno[2,3-b]pyridin-6-yl}-acetic acid ethyl ester | 7.19min, 404 (ES+, 100%, [M+H]) |
| 42 | 2-{3-Phenyl-4-[(pyridin-2-ylmethyl)-amino]-thieno[2,3-b]pyridin-6-yl}-malonic acid diethyl ester | 7.59 min, 476 (ES+, 100%, [M+H]) |
| 43 | 2-{3-Phenyl-4-[(pyridin-2-ylmethyl)-amino]-thieno[2,3-b]pyridin-6-yl}-ethanol | 6.35 min, 362 (ES+, 100%, [M+H]) |
| 44 | 2-3-Phenyl-4-[(pyridin-2-ylmethyl)-amino]-thieno[2,3-b]pyridin-6-yl}-propane-1,3-diol | 6.09 min, 392 (ES+, 100%, [M+H]) |

### Example 46

### Kvl.3 Autopatch Electrophysiology Method

Cells stably transfected with cDNA for human Kvl.3 (in pcDNA3.1) were grown in Ex-cell 302 serum-free medium for CHO cells, supplemented with 10µl/ml [100x] glutamine, 500µg/ml G418, and 1% HT supplement (50x, hypoxanthine and thymidine). Compounds were tested on these cells using the AutoPatch technology in whole cell mode.

The external bathing solution contained (in mM): 150 NaCl, 10 KCl, 1 MgCl₂, 3 CaCl₂, 10 HEPES, pH 7.4 with NaOH. Patch pipettes were filled with an electrode solution of composition (in mM): 100 K-Gluconate, 20 KCl, 1 MgCl₂, 1 CaCl₂, 10 HEPES, 11 EGTA, 5 ATP-Na₂, 2 Glutathione pH 7.2 with KOH.

Compounds were dissolved in DMSO (100%) and made up in the external bather at a concentration of 1µM. All experiments were conducted at room temperature (22-24°C).

A cell suspension (10ml), with a density of 100,000 cells/ml, was aliquoted into a 15ml centrifuge tube and transferred to an incubator (37°C, 5% CO₂) for approximately one hour before use. Following 60 min incubation, a tube was taken and centrifuged at 1000 rpm for 4 mins at room temperature. 9.5ml supernatant was thence discarded, leaving a cell pellet at the bottom of the tube. The pellet was then resuspended using 100 µl of cold (4°C), filtered (0.22µm), 0.2 % BSA/bather solution (0.02g BSA/10ml bather). The bottom of the tube was manually agitated gently until the solution became cloudy with cells. The 100µl cell resuspension solution was then stored on the bench at 4°C (using a Peltier-based temperature control device) until used.

A length of capillary glass (1B150F-4, WPI) was dipped into the cell suspension solution, such that - 3cm column of fluid was taken up by capillary action. A Ag/AgCl wire was dropped into the non-dipped end of the capillary also. The outside of the solution-filled end of the capillary was then dried and the capillary was loaded into the AutoPatch™. Borosilicate glass patch pipettes (from 1.5mm OD, thin-walled filamented, GC150-TF capillary glass, Harvard) were pulled using a DMZ pipette puller (Zeitz Instruments), and were back-filled using the internal pipette solution, being careful that no bubbles remain at the tip or in the body of the pipette. Patch pipettes typically had resistances of 2.3-3.5 MΩ. Once filled, the pipette tip and a proportion of the shaft (- 15mm) were dipped into Sigmacote (Sigma). The recording pipette was then loaded into the AutoPatch™. Automated patch-clamping was initiated by the operator, but thereafter AutoPatch exe continued the experiment providing that pre-set conditions and criteria were satisfied.

Whole cell patch-clamp recordings were made using the AutoPatch™ rig, which incorporated an EPC9 or EPC10 amplifier (HEKA, Germany) under control of Pulse software (v8.54 or v8.76, HEKA, Germany), a motion controller with 2 translators (Newport, UK), valve controller (VF1) and a c-level suction device all at room temperature (22-24°C). This equipment was completely under the control of AutoPatch.exe and operator intervention was only made when there was a requirement to refill the drug reservoirs or to prevent the loss of a cell due to a technical error. Cells with an Rₛₑᵣᵢₑₛ greater than 18 MΩ were discounted from the experiment.

Qualification stages prior to perfusion and drug application ensured that the observed current met the criteria for the experiment. Only those cells with an *I*_{K} > 400 pA were used for experiments. Cells were continuously perfused with external solution at a flow rate of 1.8-2 ml/minute. The perfusion chamber had a working volume of 80-85µl and allowed for rapid exchange of drug solutions. Online analysis of the hKᵥ1.3 current during the application of compounds was performed by the AutoPatch™ software. Voltage-step protocols and analysis of data was performed as described for conventional electrophysiology.

Electrophysiology voltage-step protocols and analysis of data was performed as follows. Data was sampled at 5kHz, and filtered with a -3dB bandwidth of 2.5kHz. Cells were held at a voltage of -80mV. Currents were evoked by a voltage step to +30mV for 500ms in duration every 10s. Currents were analysed using Pulsefit software (v8.54 or v8.76, HEKA, Germany), with the total charge measured during the whole of voltage step. All other plots were produced using Igor Pro (WaveMetrics).

### Example 47

### Kv1.3 Conventional Whole Cell Patch Electrophysiology Method

Cells stably transfected with cDNA for human Kv1.3 (in pcDNA3.1) were grown in Ex-cell 302 serum-free medium for CHO cells, supplemented with 10µl/ml [100x] glutamine, 500µg/ml G418, and 1% HT supplement (50x, hypoxanthine and thymidine). Media containing 10% fetal clone II calf serum (Hyclone) was added to cause cell adhesion. Compounds were tested on these cells using conventional electrophysiology equipment in whole cell mode.

The external bathing solution contained (in mM): 150 NaCl, 10 Kcl, 1 MgCl₂, 3 CaCl₂, 10 HEPES, pH 7.4 with NaOH. Patch pipettes were filled with an electrode solution of composition (in mM): 100 K-Gluconate, 20 KCl, 1 MgCl₂, 1 CaCl₂, 10 HEPES, 11 EGTA, 5 ATP-Na₂, 2 Glutathione pH 7.2 with KOH.

Compounds were dissolved in DMSO (100%) and made up in the external bather at a concentration of 1µM. All experiments were conducted at room temperature (22-24°C).

Cells were seeded onto 35mm plastic culture dishes at varying densities and allowed to adhere for at least 4h before use. Borosilicate glass patch pipettes (from 1.5mm OD, thin-walled filamented, GC150-TF capillary glass, Harvard) were pulled using a Narishege two stage pipette puller and backed filled with internal solution. Patch pipettes typically had resistances of 3.5-4.5 MΩ.

Whole cell patch-clamp recordings were made using a conventional patch clamp rig, which incorporated an EPC9 or EPC10 amplifier (HEKA, Germany) under control of Pulse software (v8.54 or v8.76, HEKA, Germany. Cells were patched manually and after obtaining the whole cell configuration, drug delivery and experimental parameters were controlled via the AutoPatch™ software. Cells with an Rₛₑᵣᵢₑₛ greater than 18 MΩ were discounted from the experiment.

Qualification stages prior to drug application ensured that the observed current met the criteria for the experiment. Only those cells with an I_{K} > 400 pA were used for experiments. Cells were continuously perfused with external solution at a flow rate of 0.5 ml/minute. Online analysis of the hKᵥ1.3 current during the application of compounds was performed by the AutoPatch™ software. Voltage-step protocols and analysis of data was performed using pulsefit (HEKA, Germany).

Electrophysiology voltage-step protocols and analysis of data was performed as follows. Data was sampled at 5kHz, and filtered with a -3dB bandwidth of 2.5kHz. Cells were held at a voltage of -80mV. Currents were evoked by a voltage step to +30mV for 500ms in duration every 10s. Currents were analysed using Pulsefit software (v8.54 or v8.76, HEKA, Germany), with the total charge measured during the whole of voltage step. All other plots were produced using Igor Pro (WaveMetrics).

### Example 48

### Kv1.5 Autopatch Electrophysiology Method

Cells stably transfected with cDNA for human Kv1.5 (in pEF6::VA-His-TOPO) were grown in Ex-cell 302 serum-free medium for CHO cells, supplemented with 10µl/ml [100x] glutamine, 5µg/ml blasticidin and 1% HT supplement (50x, hypoxanthine and thymidine). Compounds were tested on these cells using the AutoPatch technology in whole cell mode.

The external bathing solution contained (in mM): 150 NaCl, 10 KCl, 1 MgCl₂, 3 CaCl₂, 10 HEPES, pH 7.4 with NaOH. Patch pipettes were filled with an electrode solution of composition (in mM): 160 KCl, 0.5 MgCkl₂, 10 HEPES, 1 EGTA, pH 7.4 with KOH.

Compounds were dissolved in DMSO (100%) and made up in the external bather at a concentration of 1µM. All experiments were conducted at room temperature (22-24°C).

A cell suspension (10ml), with a density of 100,000 cells/ml, was aliquoted into a 15ml centrifuge tube and transferred to an incubator (37°C, 5% CO₂) for approximately one hour before use. Following 60 min incubation, a tube was taken and centrifuged at 1000 rpm for 4 mins at room temperature. 9.5ml supernatant was thence discarded, leaving a cell pellet at the bottom of the tube. The pellet was then resuspended using 100 µl of cold (4°C), filtered (0.22µm), 0.2 % BSA/bather solution (0.02g BSA/10ml bather). The bottom of the tube was manually agitated gently until the solution became cloudy with cells. The 100µl cell resuspension solution was then stored on the bench at 4°C (using a Peltier-based temperature control device) until used.

A length of capillary glass (1B150F-4, WPI) was dipped into the cell suspension solution, such that - 3cm column of fluid was taken up by capillary action. A Ag/AgCl wire was dropped into the non-dipped end of the capillary also. The outside of the solution-filled end of the capillary was then dried and the capillary was loaded into the AutoPatch™. Borosilicate glass patch pipettes (from 1.5mm OD, thin-walled filamented, GC150-TF capillary glass, Harvard) were pulled using a DMZ pipette puller (Zeitz Instruments), and were back-filled using the internal pipette solution, being careful that no bubbles remain at the tip or in the body of the pipette. Patch pipettes typically had resistances of 2.3-3.5 MΩ. Once filled, the pipette tip and a proportion of the shaft (- 15mm) were dipped into Sigmacote (Sigma). The recording pipette was then loaded into the AutoPatch™. Automated patch-clamping was initiated by the operator, but thereafter AutoPatch.exe continued the experiment providing that pre-set conditions and criteria were satisfied.

Whole cell patch-clamp recordings were made using the AutoPatch™ rig, which incorporated an EPC9 or EPC10 amplifier (HEKA, Germany) under control of Pulse software (v8.54 or v8.76, HEKA, Germany), a motion controller with 2 translators (Newport, UK), valve controller (VF1) and a c-level suction device all at room temperature (22-24°C). This equipment was completely under the control of AutoPatch.exe and operator intervention was only made when there was a requirement to refill the drug reservoirs or to prevent the loss of a cell due to a technical error. Cells with an Rₛₑᵣᵢₑₛ greater than 18 MΩ were discounted from the experiment.

Qualification stages prior to perfusion and drug application ensured that the observed current met the criteria for the experiment. Only those cells with an *I*_{K} > 500 pA were used for experiments. Cells were continuously perfused with external solution at a flow rate of 1.8-2 ml/minute. The perfusion chamber had a working volume of 80-85µl and allowed for rapid exchange of drug solutions. Online analysis of the hKᵥ1.5 current during the application of compounds was performed by the AutoPatch™ software. Voltage-step protocols and analysis of data was performed as described for conventional electrophysiology.

Electrophysiology voltage-step protocols and analysis of data was performed as follows. Data was sampled at 5kHz, and filtered with a -3dB bandwidth of 2.5kHz. Cells were held at a voltage of -80mV. Currents were evoked by a voltage step to 0mV for 1000ms in duration followed by a step to -40mV for 1000ms every 5s. Currents were analysed using Pulsefit software (v8.54 or v8.76, HEKA, Germany), with the total charge measured during 75-95% of the 0mV voltage step. All other plots were produced using Igor Pro (WaveMetrics).

### Example 49

### Kvl.5 Conventional Whole Cell Patch Electrophysiology Method

Cells stably transfected with cDNA for human Kvl.5 (in pEF6::VA-His-TOPO) were grown in Ex-cell 302 serum-free medium for CHO cells, supplemented with 10µl/ml [100x] glutamine, 5µg/ml blasticidin and 1% HT supplement (50x, hypoxanthine and thymidine). Media containing 10% fetal clone II calf serum (Hyclone) was added to cause cell adhesion. Compounds were tested on these cells using conventional electrophysiology equipment in whole cell mode.

The external bathing solution contained (in mM): 150 NaCl, 10 KCl, 1 MgCl₂, 3 CaCl₂, 10 HEPES, pH 7.4 with NaOH. Patch pipettes were filled with an electrode solution of composition (in mM): 160 KCl, 0.5 MgCl₂, 10 HEPES, 1 EGTA, pH 7.4 with KOH.

Compounds were dissolved in DMSO (100%) and made up in the external bather at a concentration of 1µM.All experiments were conducted at room temperature (22-24°C).

Cells were seeded onto 35mm plastic culture dishes at varying densities and allowed to adhere for at least 4h before use. Borosilicate glass patch pipettes (from 1.5mm OD, thin-walled filamented, GC150-TF capillary glass, Harvard) were pulled using a Narishege two stage pipette puller and backed filled with internal solution. Patch pipettes typically had resistances of 3.5-4.5 MΩ.

Whole cell patch-clamp recordings were made using a conventional patch clamp rig, which incorporated an EPC9 or EPC10 amplifier (HEKA, Germany) under control of Pulse software (v8.54 or v8.76, HEKA, Germany. Cells were patched manually and after obtaining the whole cell configuration, drug delivery and experimental parameters were controlled via the AutoPatch™ software. Cells with an Rₛₑᵣᵢₑₛ greater than 18 MΩ were discounted from the experiment.

Qualification stages prior to drug application ensured that the observed current met the criteria for the experiment. Only those cells with an I_{K} > 400 pA were used for experiments. Cells were continuously perfused with external solution at a flow rate of 0.5 ml/minute. Online analysis of the hKᵥ1.3 current during the application of compounds was performed by the AutoPatch™ software. Voltage-step protocols and analysis of data was performed using pulsefit (HEKA, Germany).

Electrophysiology voltage-step protocols and analysis of data was performed as follows. Data was sampled at 5kHz, and filtered with a -3dB bandwidth of 2.5kHz. Cells were held at a voltage of -80mV. Currents were evoked by a voltage step to 0mV for 1000ms in duration followed by a step to -40mV for 1000ms every 5s. Currents were analysed using Pulsefit software (v8.54 or v8.76, HEKA, Germany), with the total charge measured during 75-95% of the 0mV voltage step. All other plots were produced using Igor Pro (WaveMetrics).

### Example 50

### Representative biological data is presented below:

| Example | Kv1.3 % Inhibition at 1µM | Kv1.5 % Inhibition at 1µM |
|---|---|---|
| 24 | 93 | 79 |
| 25 | 82 | 81 |
| 19 | 72 | 88 |
| 20 | 99 | 93 |
| 36 | 32 | 20 |
| 26 | 45 | 91 |
| 28 | 20 | 21 |
| 27 | 72 | 55 |
| 29 | 62 | 31 |
| 21 | 71 | 59 |
| 22 | 78 | 95 |
| 37 | 30 | 36 |
| 35 | 45 | 62 |
| 43 | 20 | 94 |
| 44 | 8 | 82 |

### Abbreviations

- Kv₍ᵤᵣ₎: Cardiac Ultrarapid Delayed Rectifier
- CHO: Chinese Hamster Ovary Cells
- DMEM: Dulbecco's Modified Eagle media
- FCS: Fetal Calf Serum
- EBSS: Earls Balanced Salt Solution
- WCPC: Whole-Cell Patch-Clamp
- DCM: Dichloromethane
- NMP: N-methylpyrrolidinone
- HCL: Hydrochloric acid
- Na₂SO₄: Sodium Sulphate
- DME: 1,2-Dimethoxyethane
- EAE: Experimental autoimmune encephalomyelitis
- EBSS: Earls Balanced Salt Solution
- EtOAc: Ethyl acetate
- EtOH: Ethanol
- GLUT4: Insulin-regulated glucose transporter
- HT: Hydroxytryptamine
- MgSO4: Magnesium sulfate
- MS: Multiple sclerosis
- T_{CM}: Central memory T cell
- T_{EM}: Effector memory T cell

### References

Herbert, "General principles of the structure of ion channels", Am. J. Med, 104, 87-98, 1998.
Armstrong & Hille, "Voltage-gated ion channels and electrical excitability", Neuron, 20, 371-380, 1998.
Gutman GA et al., International Union of Pharmacology. XLI. Compendium of voltage-gated ion channels: potassium channels. Pharmacol Rev. 2003 Dec; 55(4):583-6.
Shieh et al. "Potassium channels: molecular defects, diseases, and therapeutic opportunities", Pharmacol Rev, 52(4), 557-594, 2000.
Ford et al. "Potassium Channels: Gene Family, Therapeutic Relevance, High-Throughput Screening Technologies and Drug Discovery", Prog Drug Res, 58, 133-168, 2002.
Marban "Cardiac channelopalthies", Nature, 415, 213-218, 213-218, 2002.
Brendel and Peukert 'Blockers of the Kv1.5 Channel for the Treatment of Atrial Arrhythmias', Expert Opinion in Therapeutic Patents, 12 (11), 1589-1598, 2002.
Wang et al., "Sustained depolarization-induced outward current in human atrial myocytes. Evidence for a novel delayed rectifier K+ current similar to Kv1.5 cloned channel currents", Circ Res, 73, 1061-1076, 1993.
Fedida et al., "Identity of a novel delayed rectifier current from human heart with a cloned K+ channel current", Circ Res , 73, 210-216, 1993.
Feng et al., "Antisense oligodeoxynucleotides directed against Kv1.5 mRNA specifically inhibit ultrarapid delayed rectifier K+ current in cultured adult human atrial myocytes", Circ Res, 80, 572-579, 1997.
Amos et al., "Differences between outward currents of human atrial and subepicardial ventricular myocytes", J Physiol , 491, 31-50, 1996.
Li et al., "Evidence for two components of delayed rectifier K+ current in human ventricular myocytes", Circ Res, 78, 689-696, 1996.
Nattel, 'Therapeutic implications of atrial fibrillation mechanisms: can mechanistic insights be used to improve AF management?' Cardiovascular Research, Volume 54, (2), 347-360, 2002.
Courtemanche et al., "Ionic targets for drug therapy and atrial fibrillation-induced electrical remodeling: insights from a mathematical model", Cardiovasc Res, 42(2), 477-489, 1999.
Nattel et al., "Cardiac ultrarapid delayed rectifiers: a novel potassium current family of functional similarity and molecular diversity", Cell Physiol Biochem, 9(4-5), 217-226, 1999.
Knobloch K. et al. Electrophysiological and antiarrhythmic effects of the novel I(Kur) channel blockers, S9947 and S20951, on left vs. right pig atrium in vivo in comparison with the I(Kr) blockers dofetilide, azimilide, d,l-sotalol and ibutilide. Naunyn Schmiedebergs Arch Pharmacol. Nov;366(5):482-7, 2002.
Wirth KJ et al.,Atrial effects of the novel K(+)-channel-blocker AVE0118 in anesthetized pigs. Cardiovasc Res. Nov 1;60 (2):298-306, 2003.
Colatsky et al., "Channel specificity in antiarrhythmic drug action. Mechanism of potassium channel block and its role in suppressing and aggravating cardiac arrhythmias", Circulation, 82(6), 2235-2242, 1990.
Feng et al., "Effects of class III antiarrhythmic drugs on transient outward and ultra-rapid delayed rectifier currents in human atrial myocytes", J Pharmacol Exp Ther, 281(1), 384-392,1997.
Wang et al., "Effects of flecainide, quinidine, and 4-aminopyridine on transient outward and ultrarapid delayed rectifier currents in human atrial myocytes", J Pharmacol, 272(1), 184-196, 1995.
Malayev et al., "Mechanism of clofilium block of the human Kv1.5 delayed rectifier potassium channel", Mol Pharmaco, 147(1), 198-205, 1995.
Godreau et al., "Mechanisms of action of antiarrhythmic agent bertosamil on hKv1.5 channels and outward potassium current in human atrial myocytes", J Pharmacol Exp Ther 300(2), 612-620, 2002.
Matsuda et al., "Inhibition by a novel anti-arrhythmic agent, NIP-142, of cloned human cardiac K+channel Kv1.5 current". Life Sci, 68, 2017-2024, 2001.
Bachmann et al., "Characterization of a novel Kv1.5 channel blocker in Xenopus oocytes, CHO cells, human and rat cardiomyocytes", Naunyn Schmiedebergs Arch Pharmacol, 364(5), 472-478, 2001.
Peukert S, et al., Identification, synthesis, and activity of novel blockers of the voltage-gated potassium channel Kv1.5. J Med Chem. Feb 13;46 (4):486-98, 2003.
Xu & Xu, "The expression of arrhythmic related genes on Xenopus oocytes for evaluation of class III antiarrhythmic drugs from ocean active material", Yi Chuan Xue Bao, 27 (3), 195-201, 2000.
Page and Rodin, 'Drug Therapy for Atrial Fibrillation: Where do we go from here?', Nature Reviews Drug Discovery, 4, 899, 2005.
Xu J et al., "The Voltage-Gated Potassium Channel Kv1.3 Regulates Peripheral Insulin Sensitivity", Proc. Natl. Acad. Sci. USA., 101 (9), 3112-3117, 2004.
Desir GV, "Kv1.3 Potassium Channel Blockade as an Approach to Insulin Resistance", Expert Opin. Ther. Targets, 9 (3), 571-579, 2005.
Leonard et al., "Selective Blockers of Voltage Gated K+ Channels Depolarized Human T Lymphocytes: Mechanism of the Antiproliferative Effects of Charybdotoxin", Proc. Natl. Acad. Sci. USA, 89, 10094, 1992.
   (a) Wulff H et al., "Potassium Channels as Therapeutic Targets for Autoimmune Disorders", Curr. Opin. Drg Disov. Dev., 6, 640-647, 2003.
      Wulff H et al., "K+ Channel Expression During B Cell Differentiation: Implications for Immunomodulation and Autoimmunity", J Immunol., 173, 776-786, 2004.
      Beeton C et al., "A Novel Fluorescent Toxin to Detect and Investigate Kv1.3 Channel Up-regulation in Chronically Activated T Lymphocytes", J. Biol. Chem., 278 (11), 9928-9937,2003.
   (b) Wulff H et al., "The Voltage-gated Kv1.3 K+ Channel in Effector Memory T Cells as New Targets for MS", J. Clin. Invest., 111, 1703-1713, 2003. O'Connor KC et al., "The Neuroimmunology of Multiple Sclerosis: Possible Roles of T and B Lymphocytes in Immunopathogenesis", J. Clin. Immunol., 21, 81, 2001.
   Beeton C et al., "Selective Blockade of T Lymphocyte K+ Channels Ameliorates Experimental Autoimmune Encephalomyelitis, a Model for Multiple Sclerosis", Natl. Acad. Sci. USA, 98 (24), 13942-13947, 2001.
   Valverde P et al., "Potassium Channel-blockers as Therapeutic Agents to Interfere with Bone Resorption of Periodontal Disease", J. Dent. Res., 84 (6), 488-499, 2005.
   Friedrich M et al., "Flow Cytometric Characterization of Lesional T Cells in Psoriasis: Intracellular Cytokine and Surface Antigen Expression Indicates an Activated, Memory Efector/Type 1 Immunophenotype", Arch. Dermatol. Res., 292, 519, 2000.
   Yoon JW et al., "Cellular and Molecular Pathogenic Mechanisms of Insulin-Dependent Diabetes Mellitus", Ann. NY Acad. Sci., 928, 200, 2001.
   Viglietta et al., "GAD65-Reactive T Cells are Activated in Patients with Autoimmune Type la Diabetes", J. Clin. Invest., 109, 895,2002.
   Yamashita K et al., "Severe Chronic Graft-versus-host Disease is Characterized by a Preponderance of CD4+ Effector Memory Cells Relative to Central Memory Cells", Blood, 103, 3986-3988, 2004.
   Shah K et al., "Immunosuppressive Effects of a Kv1.3 Inhibitor", Cell. Immunol., 221, 100-106, 2003.
   Nguyen A et al., "Novel Nonpeptide Agents Potently Block the C-Type Inactivated Conformation of Kv1.3 and Suppress T Cell Activation", Mol. Pharmacol., 50, 1672-1679, 1996.
   Hanson DC et al., "UK-78,282, a Novel Piperidine Compound That Potently Blocks the Kv1.3 Voltage-Gated Potassium Channel and Inhibits Human T Cell Activation", Br. J. Pharmacol., 126, 1707-1716, 1999.
   Felix JP et al., "Identification and Biochemical Characterization of a Novel Norterpene Inhibitor of the Human Lymphocyte Voltage-Gated Potassium Channel, Kv1.3", Biochemistry, 38 (16), 4922-4930, 1999.
   Baell JB et al., "Khellinone Derivatives as Blockers of he Voltage-Gated Potassium Channel Kv1.3: Synthesis and Immunosuppressive Activity" J. Med. Chem., 47, 2326-2336, 2004.
   Wulff H et al., "Alkoxypsoralens, Novel Nonpeptide Blockers of Shaker-Type K+ Channels: Synthesis and Photoreactivity", J. Med. Chem., 41, 4542-4549, 1998.
   Vennekamp J et al., "Kv1.3-Blocking 5-Phenylalkoxypsoralens: A New Class for Immunomodulators", Mol. Pharmacol., 65, 364-1374,2004.
   Schmitz A et al., "Design of PAP-1, a Selective Small Molecule Kv1.3 Blocker, for the Suppression of Effector Memory T Cells in Autoimmune Diseases", Mol. Pharmacol., 15669, 2005.
   Gilis, P.M. et al, Synthesis and antibacterial evaluation of 4, 7-dihydro-4-oxothieno[2,3-b] pyridine-5-carboxylic acids. Eur. J. Med. Chem. Chim. Ther.; 13; 265-269. 1978.
   Abdelrazek et al, Synthesis of novel thieno[2,3-d] pyrimidine, thieno[2,3-b]pyridine and thiazolo[3,2-a]thieno[2,3-d]pyrimidine derovatives and their effect on the production of mycotoxins, Arch. Pharm. (Weinheim Ger.); 325 (5) 301-306, 1992.
   Suzuki et al, Synthesis and biological evaluations of condensed pyridine and condensed pyrimidine-based HMG-CoA Reductase inhibitors, Bioorg. Med. Chem. Lett; 11 (10); 1285-1288, 2001.
   Marco et al, Synthesis and acetylcholesterase/butyrylcholinesterase inhibition activity of of 4-amino-2,3-diaryl-5,6,7,8-tetrahydrofuro(and thieno)[2,3-b]-quinolines, and 4-amino-5,6,7,8,9-pentahydro-2,3-diphenylcyclohepta[e]furo(and thieno)-[2,3-b] pyridines. Arch. Pharm. (Weinheim Ger.) 335; (7) 347-353, 2002.
   Munchof et al., Design and SAR of thienopyrimidine and thienopyridine inhibitors of VEGFR-2 kinase activity. Bioorganic & Medicinal Chemistry Letters, 14 (1), 21-24, 2004.
   Barker et al , Thienopyridines Part 6. Synthesis and nucleophilic substitution of some chlorothieno[2,3b]pyridine derivatives and comparison with the analogous quinoline compounds. J. Chem Res. (Miniprint), 2501-2509, 1998.
   Charvát et al., Diethyl Acetonedicarboxylate - a Precursor for the Synthesis of new Substituted 4-Aminoquinolines and Fused 4-Aminopyridines. Monatsheft. Chem. 126, 333-340, 1995.
   Gewald et al, Synthesen von 4-Amino-thieno[2,3-b]pyridinen, Monatsheft. Chem. 110, 1189-1196, 1979.
   Boschelli et al, Identification of 7-Phenylaminothieno- [3,2-b]pyridine-6-carbonitriles as a New class of Src Kinase Inhibitors, J. Med. Chem. 47, 6666-6668, 2004.
   Meadows et al , Effect of SB-205384 on the decay of GABA-activated chloride currents in granule cells cultured from rat cerebellum, British Journal of Pharmacology, 121 (7), 1334-1338, 1997.

## Claims

1. A compound of the formula: 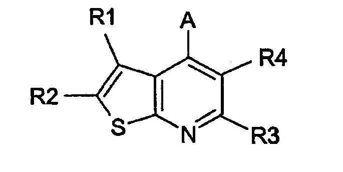 wherein
R1 is C₆-C₁₀ aryl optionally substituted by cyano, halogen, nitro, trifluoromethyl, alkyl, alkylthio, alkoxy, NR9R10, CO₂R7, C(O)NR9R10, NC(O)R7, SO₂NR9R10 or hydroxyl , 5 to 10 membered heteroaryl, optionally substituted by cyano, nitro, halogen, alkyl, alkylthio, alkoxy, NR9R10, CO₂R7, C(O)NR9R10, NC(O)R7, SO₂NR9R10 or hydroxyl or C₃-C₆ cycloalkyl, optionally substituted by halogen, cyano, nitro, NR9R10, alkoxy, hydroxyl, unsubstituted heteroaryl, CO₂R7, C(O)NR9R10, NC(O)R7 or SO₂NR9R10 ;
R2 is H, alkyl, nitro, CO₂R7 CONR5R6 or halo;
R3 and R4 are H, NR5R6, NC(O)R7, halo, trifluromethyl, alkyl, CONR5R6, CO₂R7, nitrile or alkoxy;
R5 and R6 may be the same or different and may be H, alkyl, aryl, heteroaryl or cycloalkyl,; or R5 and R6 may together form a saturated, unsaturated or partially saturated 4 to 7 member ring, wherein said ring may optionally comprise one or more further heteroatoms selected from N. O or S;
R7 is H or alkyl;
A is halo, or a group of formula X-L-Y;
X is O, S or NR8;
R8 is H or alkyl;
L is (CH₂)ₙ, where n is 0, 1, 2, 3 or 4; and
Y is aryl, a heterocyclic group, alkyl, alkenyl or cycloalkyl;
Wherein R9 and R10 are selected from H, unsubstituted alkyl, unsubstituted aryl, unsubstituted heteroaryl, unsubstituted cycloalkyl, aminoethyl, methylaminoethyl, dimethylaminoethyl, hydroxyethyl, alkoxyethyl or R9 and R10 may together form a saturated, unsaturated or partially saturated 4 to 7 member ring;
the products of mono- and di-oxidation of sulphur and/or mono-oxidation of nitrogen moieties in compounds of formula I ;
or a pharmaceutically acceptable salt thereof.

2. A compound as claimed in claim 1 wherein R1 is C₆-C₁₀ aryl, C₃-C₆ cycloalkyl or 5 to 10 membered heteroaryl; R2 is H or alkyl; R3 and R4 are H, NR5R6, alkoxy or alkyl; X is O or NR8; R8 is H or alkyl; n is 0, 1, 2, 3 or 4 and Y is alkyl, cycloalkyl, aryl or heteroaryl.

3. A compound as claimed in claim 2 wherein R1 is C₆-C₁₀ aryl or 5 to 10 membered heteroaryl, R2 is H or alkyl, R3 and R4 are H, NR5R6, alkoxy or alkyl, X is O or NR8, R8 is H, n is 0, 1 or 2 and Y is aryl or heteroaryl.

4. A compound as claimed in claim 3 which is:
(3-Phenyl-thieno[2,3-b]pyridin-4-yl)-pyridin-2-ylmethyl-amine,
2-{3-Phenyl-4-[(pyridin-2-ylmethyl)-amino]-thieno[2,3-b]pyridin-6-ylamino}-ethanol,
2-((2-Hydroxy-ethyl)-{3-phenyl-4-[(pyridin-2-ylmethyl)-amino]-thieno[2,3-b]pyridin-6-yl}-amino)-ethanol,
(6-Chloro-3-phenyl-thieno[2,3-b]pyridin-4-yl)-pyridin-2-ylmethyl-amine,
[3-(4-Fluoro-phenyl)-thieno[2,3-b]pyridin-4-yl]-pyriin-2-ylmethyl-amine,
2-[{3-(4-Fluoro-phenyl)-4-[(pyridin-2-ylmethyl)-amino]-thieno[2,3-b]pyridin-6-yl}-(2-hydroxy-ethyl)-amino]-ethanol, 2-[{3-(4-Fluoro-phenyl)-4-[(6-methyl-pyridin-2-ylmethyl)-amino]-thieno[2,3-b]pyridin-6-yl}-(2-hydroxy-ethyl)-amino]-ethanol,
2-{3-(4-Fluoro-phenyl)-4-[(pyridin-2-ylmethyl)-amino]-thieno[2,3-b]pyridin-6-ylamino} -ethanol,
2-{3-(4-Fluoro-phenyl)-4-[(6-methyl-pyridin-2-ylmethyl)-amino]-thieno[2,3-b]pyridin-6-ylainino}-ethanol,
2-{2-Methyl-3-phenyl-4-[(pyridin-2-ylmethyl)-amino]-thieno[2,3-b]pyridin-6-ylamino}- ethanol,
[6-Chloro-3-(4-fluoro-phenyl)-thieno[2,3-b]pyridin-4-yl]-pyridin-2-ylmethyl-amine, [6-Chloro-3-(4-fluoro-phenyl)-thieno[2,3-b]pyridin-4-yl]-(6-methyl-pyridin-2-ylmethyl)-amine,
[3-(4-Fluoro-phenyl)-thieno[2,3-b]pyridin-4-yl]-(6-methyl-pyridin-2-ylmethyl)-amine,
2-{3-Phenyl-4-[(pyridin-2-ylmethyl)-amino]-thieno[2,3-b]pyridin-6-yl}-propane-1,3-diol (4-Chloro-3-phenyl-thieno[2,3-b]pyridin-6-yl)pyridin-2-ylmethyl-amine.

5. A compound of the formula: 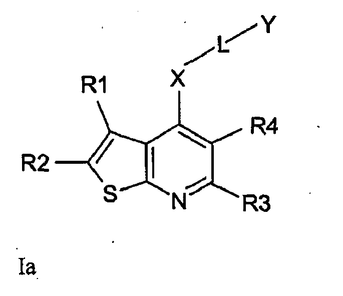 wherein:
R1 is C₆-C₁₀ aryl optionally substituted by cyano, halogen, nitro, trifluoromethyl, alkyl, alkylthio, alkoxy, NR9R10, CO₂R7, C(O)NR9R10, NC(O)R7, SO₂NR9R10 or hydroxyl , 5 to 10 membered heteroaryl, optionally substituted by cyano, nitro, halogen, alkyl, alkylthio, alkoxy, NR9R10, CO₂R7, C(O)NR9R10, NC(O)R7, SO₂NR9R10 or hydroxyl or C₃-C₆ cycloalkyl, optionally substituted by halogen, cyano, nitro, NR9R10, alkoxy, hydroxyl, unsubstituted heteroaryl, CO₂R7, C(O)NR9R10, NC(O)R7 or SO₂NR9R10 ;
R2 is H, alkyl, nitro, CO₂R7, CONR5R6 or halo;
R3 and R4 are H, NR5R6, NC(O)R7, halo, trifluromethyl, alkyl, CONRSR6, CO₂R7, nitrile or alkoxy;
R5 and R6 may be the same or different and may be H, alkyl, aryl, heteroaryl or cycloalkyl; or R5 and R6 may together form a saturated, unsaturated or partially saturated 4 to 7 member ring, wherein said ring may optionally comprise one or more further heteroatoms selected from N, O or S;
R7 is H or alkyl;
X is O, S or NR8;
R8 is H or alkyl;
L is (CH₂)ₙ where n is 1,2 or 3; and
Y is aryl, a heterocyclic group, alkyl, alkenyl or cycloalkyl;
Wherein R9 and R10 are selected from H, unsubstituted alkyl, unsubstituted aryl, unsubstituted heteroaryl, unsubstituted cycloalkyl, aminoethyl, methylaminoethyl, dimethylaminoethyl, hydroxyethyl, alkoxyethyl or R9 and R10 may together form a saturated, unsaturated or partially saturated 4 to 7 member ring;
or a pharmaceutically acceptable salt thereof.

6. A pharmaceutical composition comprising at least one compound as claimed in any one of claims 1 to 5 optionally together with one or more pharmaceutically acceptable excipients, diluents and/or carriers.

7. The use of a compound as defined in any one of claims 1 to 5 in the manufacture of a medicament for use in potassium channel inhibition.

8. The use as claimed in claim 10 wherein the medicament is for use in the treatment of arrythmia.

9. The use as claimed in claim 7 wherein the medicament is for use in the treatment of type-2 diabetes mellitus, immunological disorders, including rheumatoid arthritis, type-1 diabetes, inflammatory bowel disorder and demyelinating disorders such as multiple sclerosis.

10. A compound as defined in any one of claims I to 5 for use in medicine.

## Patentansprüche

1. Verbindung der Formel: 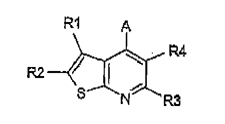 worin
R1 für C₆-C₁₀-Aryl, gegebenenfalls substituiert durch Cyano, Halogen, Nitro, Trifluormethyl, Alkyl, Alkylthio, Alkoxy, NR9R10, CO₂R7, C(O)NR9R10, NC(O)R7, SO₂NR9R10 oder Hydroxyl, 5-bis 10-gliedriges Heteroaryl, gegebenenfalls substituiert durch Cyano, Nitro, Halogen, Alkyl, Alkylthio, Alkoxy, NR9R10, CO₂R7, C(O)NR9R10, NC(O)R7, SO₂NR9R10 oder Hydroxyl, oder C₃-C₆-Cycloalkyl, gegebenenfalls substituiert durch Halogen, Cyano, Nitro, NR9R10, Alkoxy, Hydroxyl, unsubstituiertes Heteroaryl, CO₂R7, C(O)NR9R10, NC(O)R7 oder SO₂NR9R10 steht;
R2 für H, Alkyl, Nitro, CO₂R7, CONR5R6 oder Halogen steht; R3 und R4 für H, NR5R6, NC(O)R7, Halogen, Trifluormethyl, Alkyl, CONR5R6, CO₂R7, Nitril oder Alkoxy stehen;
R5 und R6 gleich oder verschieden sein können und für H, Alkyl, Aryl, Heteroaryl oder Cycloalkyl stehen können; oder R5 und R6 zusammen einen gesättigten, ungesättigten oder teilweise gesättigten 4- bis 7-gliedrigen Ring bilden können, wobei besagter Ring gegebenenfalls ein oder mehrere weitere Heteroatome, ausgewählt aus N, O oder S, umfassen kann;
R7 für H oder Alkyl steht;
A für Halogen oder eine Gruppe der Formel X-L-Y steht;
X für O, S oder NR8 steht;
R8 für H oder Alkyl steht;
L für (CH₂), steht, wo n 0, 1, 2, 3 oder 4 ist; und
Y für Aryl, eine heterocyclische Gruppe, Alkyl, Alkenyl oder Cycloalkyl steht;
worin R9 und R10 ausgewählt sind aus H, unsubstituiertem Alkyl, unsubstituiertem Aryl, unsubstituiertem Heteroaryl, unsubstituiertem Cycloalkyl, Aminoethyl, Methylaminoethyl, Dimerthylaminoethyl, Hydroxyethyl, Alkoxyethyl oder R9 und R10 zusammen einen gesättigten, ungesättigten oder teilweise gesättigten 4- bis 7-gliedrigen Ring bilden können;
die Produkte von Mono- und Dioxidation von Schwefel- und/oder Monooxidation von Stickstoffkomponenten in Verbindungen der Formel I;
oder ein pharmazeutisch annehmbares Salz davon.

2. Verbindung wie in Anspruch 1 beansprucht, worin R1 für C₆-C₁₀-Aryl, C₃-C₆-Cycloalkyl oder 5- bis 10-gliedriges Heteroaryl steht; R2 für H oder Alkyl steht; R3 und R4 für H, NR5R6, Alkoxy oder Alkyl stehen; X für O oder NR8 steht; R8 für H oder Alkyl steht; n 0, 1, 2, 3 oder 4 ist und Y für Alkyl, Cycloalkyl, Aryl oder Heteroaryl steht.

3. Verbindung wie in Anspruch 2 beansprucht, worin R1 für C₆-C₁₀-Aryl oder 5- bis 10-gliedriges Heteroaryl steht, R2 für H oder Alkyl steht, R3 und R4 für H, NR5R6, Alkoxy oder Alkyl stehen, X für O oder NR8 steht, R8 für H steht, n 0, 1 oder 2 ist und Y für Aryl oder Heteroaryl steht.

4. Verbindung wie in Anspruch 3 beansprucht, welche ist:
(3-Phenyl-thieno[2,3-b]pyridin-4-yl)-pyridin-2-ylmethyl-amin,
2-{3-Phenyl-4-[(pyridin-2-ylmethyl)-amino}-thieno[2,3-b]pyridin-6-ylamino}-ethanol,
2-((2-Hydroxy-ethyl)-{3-phenyl-4-[(pyridin-2-ylmethyl)-amino]-thieno[2,3-b]pyridin-6-yl}-amino)-ethanol,
(6-Chlor-3-phenyl-thieno[2,3-b]pyridin-4-yl)-pyridin-2-ylmethyl-amin,
[3-(4-Fluor-phenyl)-thieno[2,3-b]pyridin-4-yl]-pyridin-2-ylmethyl-amin,
2-[{3-(4-Fluor-phenyl)-4-[(pyridin-2-ylmethyl)-amino]-thieno[2,3-b]pyridin-6-yl}-(2-hydroxy-ethyl)-amino]-ethanol,
2-[{3-(4-Fluor-phenyl)-4-[(6-methyl-pyridin-2-ylmethyl)-amino]-thieno[2,3-b]pyridin-6-yl}-(2-hydroxy-ethyl)-amino]-ethanol,
2-{3-(4-Fluor-phenyl)-4-[(pyridin-2-ylmethyl)-amino]-thieno[2,3-b]pyridin-6-ylamino}-ethanol,
2-{3-(4-Fluor-phenyl)-4-[(6-methyl-pyridin-2-ylmethyl)-amino]-thieno[2,3-b]pyridin-6-ylamino}-ethanol,
2-{2-Methyl-3-phenyl-4-[(pyridin-2-ylmethyl)-amino]-thieno[2,3-b]pyridin-6-ylamino}-ethanol,
[6-Chlor-3-(4-fluor-phenyl)-thieno[2,3-b]pyridin-4-yl]-pyridin-2-ylmethyl-amin,
[6-Chlor-3-(4-fluor-phenyl)-thieno[2,3-b]pyridin-4-yl]-(6-methyl-pyridin-2-ylmethyl)-amin,
[3-(4-Fluor-phenyl)-thieno[2,3-b]pyridin-4-yl]-(6-methyl-pyridin-2-ylmethyl)-amin,
2-{3-Phenyl-9-[(pyridin-2-ylmethyl)-amino]-thieno[2,3-b]pyridin-6-yl}-propan-1,3-diol
(4-Chlor-3-phenyl-thieno[2,3-b]pyridin-6-yl)-pyridin-2-ylmethyl-amin.

5. Verbindung der Formel: 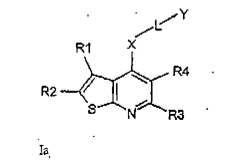 worin:
R1 für C₆-C₁₀-Aryl, gegebenenfalls substituiert durch Cyano, Halogen, Nitro, Trifluormethyl, Alkyl, Alkylthio, Alkoxy, NR9R10, CO₂R7, C(O)NR9R10, NC(O)R7, SO₂NR9R10 oder Hydroxyl, 5-bis 10-gliedriges Heteroaryl, gegebenenfalls substituiert durch Cyano, Nitro, Halogen, Alkyl, Alkylthio, Alkoxy, NR9R10, CO₂R7, C(O)NR9R10, NC(O)R7, SO₂NR9R10 oder Hydroxyl, oder C₃-C₆-Cycloalkyl, gegebenenfalls substituiert durch Halogen, Cyano, Nitro, NR9R10, Alkoxy, Hydroxyl, unsubstituiertes Heteroaryl, CO₂R7, C(O)NR9R10, NC(O)R7 oder SO₂NR9R10 steht;
R2 für H, Alkyl, Nitro, CO₂R7, CONR5R6 oder Halogen steht;
R3 und R4 für H, NR5R6, NC(O)R7, Halogen, Trifluormethyl, Alkyl, CONRSR6, CO₂R7, Nitril oder Alkoxy stehen;
R5 und R6 gleich oder verschieden sein können und für H, Alkyl, Aryl, Heteroaryl oder Cycloalkyl stehen können; oder R5 und R6 zusammen einen gesättigten, ungesättigten oder teilweise gesättigten 4- bis 7-gliedrigen Ring bilden können, wobei besagter Ring gegebenenfalls ein oder mehrere weitere Heteroatome, ausgewählt aus N, O oder S, umfassen kann;
R7 für H oder Alkyl steht;
X für 0, S oder NR8 steht;
R8 für H oder Alkyl steht;
L für (CH₂)ₙ steht, wo n 1, 2 oder 3 ist; und
Y für Aryl, eine heterocyclische Gruppe, Alkyl, Alkenyl oder Cycloalkyl steht;
worin R9 und R10 ausgewählt sind aus H, unsubstituiertem Alkyl, unsubstituiertem Aryl, unsubstituiertem Heteroaryl, unsubstituiertem Cycloalkyl, Aminoethyl. Methylaminoethyl, Dimethylaminoethyl, Hydroxyethyl, Alkoxyethyl oder R9 und R10 zusammen einen gesättigten, ungesättigten oder teilweise gesättigten 4- bis 7-gliedrigen Ring bilden können;
oder ein pharmazeutisch annehmbares Salz davon.

6. Pharmazeutische Zusammensetzung, welche mindestens eine Verbindung wie in einem von Ansprüchen 1 bis 5 beansprucht, gegebenenfalls zusammen mit einem oder mehreren pharmazeutisch annehmbaren Arzneimittelträgern, Verdünnungsmitteln und/oder Trägern umfasst.

7. Verwendung einer Verbindung wie in einem von Ansprüchen 1 bis 5 definiert in der Herstellung eines Medikaments zur Verwendung in Kaliumkanalhemmung.

8. Verwendung wie in Anspruch 10 beansprucht, wobei das Medikament zur Verwendung in der Behandlung von Arrhythmie ist.

9. Verwendung wie in Anspruch 7 beansprucht, wobei das Medikament zur Verwendung in der Behandlung von Typ-2-Diabetes mellitus, immunologischen Störungen einschließlich rheumatoider Arthritis, Typ-1-Diabetes, entzündlicher Darmerkrankung und demyelinisierenden Störungen wie Multiple Sklerose ist.

10. Verbindung wie in einem von Ansprüchen 1 bis 5 definiert zur Verwendung in Medizin.

## Revendications

1. Composé de formule : 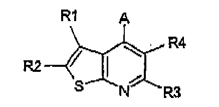 où
R1 est un aryle en C₆ à C₁₀ éventuellement substitué par un cyano, un halogène, un nitro, un trifluorométhyle, un alkyle, un alkylthio, un alcoxy, NR9R10, CO₂R7, C(O)NR9R10, NC(O)R7, SO₂NR9R10 ou un hydroxyle, un hétéroaryle de 5 à 10 membres, éventuellement substitué par un cyano, un nitro, un halogène, un alkyle, un alkylthio, un alcoxy, NR9R10, CO₂R7, C(O)NR9R10, NC(O)R7, SO₂NR9R10 ou un hydroxyle ou un cycloalkyle en C₃ à C₆, éventuellement substitué par un halogène, un cyano, un nitro, NR9R10, un alcoxy, un hydroxyle, un hétéroaryle non substitué, CO₂R7, C(O)NR9R10, NC(O)R7 ou SO₂NR9R10 ;
R2 est H, un alkyle, un nitro, CO₂R7, CONR5R6 ou un halogène ;
R3 et R4 sont H, NR5R6, NC(O)R7, un halogène, un trifluorométhyle, un alkyle, CONR5R6, Cour7, un nitrile ou un alcoxy ;
R5 et R6 peuvent être les mêmes ou différents et peuvent être H, un alkyle, un aryle, un hétéroaryle ou un cycloalkyle ; ou R5 et R6 peuvent former ensemble un cycle saturé, insaturé ou partiellement saturé de 4 à 7 membres, où ledit cycle peut facultativement comprendre un ou plusieurs hétéroatomes supplémentaires choisis parmi N, O ou S ;
R7 est H ou un alkyle ;
A est un halogène, ou un groupe de formule x-L-Y ;
X est O, S ou NR8 ;
R8 est H ou un alkyle ;
L est (CH₂)ₙ, où n est 0, 1, 2, 3 ou 4 ; et
Y est un aryle, un groupe hétérocyclique, un alkyle, un alcényle ou un cycloalkyle ;
où R9 et R10 sont choisis parmi H, un alkyle non substitué, un aryle non substitué, un hétéroaryle non substitué, un cycloalkyle non substitué, un aminoéthyle, un méthylaminoéthyle, un diméthyl-aminoéthyle, un hydroxyéthyle, un alcoxyéthyl ou R9 et R10 peuvent former ensemble un cycle saturé, insaturé ou partiellement saturé de 4 à 7 membres ;
les produits de mono- et di-oxydation des fragments de soufre et/ou de mono-oxydation des fragments d'azote dans les composés de formule I ;
ou un sel acceptable sur le plan pharmaceutique de celui-ci.

2. Composé selon la revendication 1, dans lequel R1 est un aryle en C₆ à C₁₀, un cycloalkyle en C₃ à C₆ ou un hétéroaryle de 5 à 10 membres ; R3 est H ou un alkyle ; R3 et R4 sont H, NRSR6, un alcoxy ou un alkyle ; X est O ou NR8 ; R8 est H ou un alkyle ; n est 0, 1, 2, 3 ou 4 et Y est un alkyle, un cycloalkyle, un aryle ou un hétéroaryle.

3. Composé selon la revendication 2, dans lequel R1 est un aryle en C₆ à C₁₀ ou un hétéroaryle de 5 à 10 membres, R2 est H ou un alkyle, R3 et R4 sont H, NR5R6, un alcoxy ou un alkyle, X est O ou NR8, R8 est H, n est 0, 1 ou à 2 et Y est un aryle ou un hétéroaryle.

4. Composé selon la revendication 3 qui est :
(3-phényl-thiéno[2,3,b]pyridin-4-yl)pyridin-2-ylméthyl-amine,
2-{3-phényl-4-[{pyridin-2-ylméthyl)-amino]-thiéno[2,3-b]pyridin-6-ylamino}-éthanol,
2-((2-hydroxy-éthyl)-{3-phényl-4-[(pyridin-2-ylméthyl)-amino]-thiéno[2,3-b]pyridin-6-yl}-amino)-éthanol,
(6-chloro-3-phényl-thiéno[2,3-b]pyridin-4-yl)-pyridin-2-ylméthyl-amine,
[3-(4-fluoro-phényl)-thiéno[2,3-b]pyridin-4-yl]-pyridin-2-ylméthyl-amine,
2-[{3-(4-fluoro-phényl)-4-[(pyridin-2-ylméthyl)-amino]-thiéno[2,3-b]pyridin-6-yl}-(2-hydroxy-éthyl)-aminol-éthanol,
2-[{3-(4-fluoro-phényl)-4-[(6-méthyl-pyridin-2-ylméthyl)-amino]-thiéno[2,3-b]pyridin-6-yl}-(2-hydroxy-éthyl)-amino]-éthanol,
2-{3-(4-fluoro-phényl)-4-[(pyridin-2-ylméthyl)-amino]-thiéno[2,3-b]-pyridin-6-ylamino}-éthanol,
2-{3-(4-fluoro-phényl)-4-[(6-méthyl-pyridin-2-ylméthyl)-amino]-thiéno[2,3-b]pyridin-6-ylamino}-éthanol,
2-{2-méthyl-3-phényl-4-[(pyridin-2-ylméthyl)-amino]-thiéno[2,3-b]pyridin-6-ylamino}-éthanol,
[6-chloro-3-(4-fluoro-phényl)-thiéno[2,3-b]pyridin-4-yl]-pyridin-2-ylméthyl-amine,
[6-chloro-3-(4-fluoro-phényl)-thiéno[2,3-b]pyridin-4-yl]-(6-méthyl-pyridin-2-ylméthyl)-amine,
[3-(4-fluoro-phényl)-thiéno[2,3-b]pyridin-4-yl]-(6-méthyl-pyridin-2-ylméthyl)-amine,
2-{3-phényl-4-[(pyridin-2-ylméthyl)-amino]-thiéno[2,3-b]pyridin-6-yl}-propane-1,3-diol
4-chloro-3-phényl-thiéno[2,3-b]pyridin-6-yl)-pyridin-2-ylméthyl-amine

5. Composé de formule : 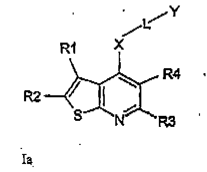 où :
R1 est un aryle en C₆ à C₁₀ éventuellement substitué par un cyano, un halogène, un nitro, un trifluorométhyle, un alkyle, un alkylthio, un alcoxy, NR9R10, CO₂R7, C(O)NR9R10, NC(O)R7, SO₂NR9R10 ou un hydroxyle, un hétéroaryle de 5 à 10 membres, éventuellement substitué par un cyano, un nitro, un halogène, un alkyle, un alkylthio, un alcoxy, NR9R10, CO₂R7, C(O)NR9R10, NC(O)R7, SO₂NR9R10 ou un hydroxyle ou un cycloalkyle en C₃ à C₆, éventuellement substitué par un halogène, un cyano, un nitro, NR9R10, un alcoxy, un hydroxyle, un hétéroaryle non substitué, CO₂R7, C(O)NR9R10, NC(O)R7 ou SO₂NR9R10 ;
R2 est H, un alkyle, un nitro, CO₂R7, CONR5R6 ou un halogène ;
R3 et R4 sont H, NR5R6, NC(O)R7, un halogène, un trifluorométhyle, un alkyle, CONR5R6, CO₂R7, un nitrile ou un alcoxy ;
R5 et R6 peuvent être les mêmes ou différents et peuvent être H, un alkyle, un aryle, un hétéroaryle ou un cycloalkyle ; ou R5 et R6 peuvent former ensemble un cycle saturé, insaturé ou partiellement saturé de 4 à 7 membres, où ledit cycle peut facultativement comprendre un ou plusieurs hétéroatomes supplémentaires choisis parmi N, O ou S ;
R7 est H ou un alkyle ;
X est O, S ou NR8 ;
R8 est H ou un alkyle ;
L est (CH₂)ₙ, où n est 1, 2 ou 3 ; et
Y est un aryle, un groupe hétérocyclique, un alkyle, un alcényle ou un cycloalkyle ;
où R9 et R10 sont choisis parmi H, un alkyle non substitué, un aryle non substitué, un hétéroaryle non substitué, un cycloalkyle non substitué, un aminoéthyle, un méthylaminoéthyle, un diméthylamino-éthyle, un hydroxyéthyle, un alcoxyéthyl ou R9 et R10 peuvent former ensemble un cycle saturé, insaturé ou partiellement saturé de 4 à 7 membres ;
ou un sel acceptable sur le plan pharmaceutique de celui-ci.

6. Composition pharmaceutique comprenant au moins un composé selon l'une quelconque des revendications 1 à 5 éventuellement conjointement avec un ou plusieurs excipients, diluants et/ou véhicules acceptables sur le plan pharmaceutique.

7. Utilisation d'un composé tel que défini dans l'une quelconque des revendications 1 à 5 dans la fabrication d'un médicament pour une utilisation dans l'inhibition des canaux potassiques.

8. Utilisation selon la revendication 10, dans laquelle le médicament est pour une utilisation dans le traitement de l'arythmie.

9. Utilisation selon la revendication 7, dans laquelle le médicament est pour une utilisation dans le traitement du diabète sucré de type 2, de troubles immunologiques, y compris la polyarthrite rhumatoïde, du diabète de type 1, d'un trouble inflammatoire intestinal et de troubles de démyélinisation tels que la sclérose en plaques.

10. Composé tel que défini dans l'une quelconque des revendications 1 à 5 pour une utilisation dans un médicament.
